# EUROPEAN PATENT SPECIFICATION

(11) **EP 2 854 805 B1**
(45) Date of publication and mention of the grant of the patent: **12.06.2019**
(21) Application number: 13721573.7
(22) Date of filing: 17.04.2013
(51) Int. Cl.: A61K 31/42, A61P 37/06

(54) **COMPOUNDS HAVING IMMUNOMODULATOR ACTIVITY**
VERBINDUNGEN MIT IMMUNMODULATORISCHER WIRKUNG
COMPOSÉS AYANT UNE ACTIVITÉ IMMUNOMODULATRICE

(30) Priority: 17.04.2012 US 201261625635 P
(43) Date of publication of application: 08.04.2015
(73) Proprietor: GeneOne Life Science Inc., Gangnam-Gu Seoul 06060 (KR)
(72) Inventor: SARDESAI, Niranjan, Blue Bell, PA 19422 (US); KIM, J.Joseph, Blue Bell, PA 19422 (US)
(74) Representative: Leonard, Thomas Charles
(86) International application number: PCT/US2013/037020
(87) International publication number: WO 2013/158792

(56) References cited:
- WO-A1-2006/097273
- WO-A1-2009/132172
- GB-A- 1 164 510
- MANGANO K ET AL: "In vitro inhibition of enterobacteria-reactive CD4+CD25- T cells and suppression of immunoinflammatory colitis in mice by the novel immunomodulatory agent VGX-1027", EUROPEAN JOURNAL OF PHARMACOLOGY, ELSEVIER SCIENCE, NL, vol. 586, no. 1-3, 31 May 2008 (2008-05-31), pages 313-321, XP022671798, ISSN: 0014-2999, DOI: 10.1016/J.EJPHAR.2008.02.017 [retrieved on 2008-02-17]
- K MANGANO ET AL: "Effects of the immunomodulator, VGX-1027, in endotoxin-induced uveitis in Lewis rats", BRITISH JOURNAL OF PHARMACOLOGY, vol. 155, no. 5, 1 November 2008 (2008-11-01), pages 722-730, XP55064840, ISSN: 0007-1188, DOI: 10.1038/bjp.2008.315
- STANISLAVA STOSIC-GRUJICIC ET AL: "A potent immunomodulatory compound, (S,R)-3-phenyl-4,5- dihydro-5-isoxasole acetic acid, prevents spontaneous and accelerated forms of autoimmune diabetes in NOD mice and inhibits the immunoinflammatory diabetes induced by multiple low doses of streptozotocin in CBA/H mice", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 320, no. 3, 1 January 2007 (2007-01-01), pages 1038-1049, XP008146433, ISSN: 0022-3565, DOI: 10.1124/JPET.106.109272 [retrieved on 2006-12-05]
- STOJANOVIC I ET AL: "In vitro, ex vivo and in vivo immunopharmacological activities of the isoxazoline compound VGX-1027: Modulation of cytokine synthesis and prevention of both organ-specific and systemic autoimmune diseases in murine models", CLINICAL IMMUNOLOGY, ACADEMIC PRESS, US, vol. 123, no. 3, 1 June 2007 (2007-06-01), pages 311-323, XP025323239, ISSN: 1521-6616, DOI: 10.1016/J.CLIM.2007.03.004 [retrieved on 2007-05-30]

## Description

### FIELD OF THE INVENTION

The present invention refers to the use of compounds for use in the treatment of autoimmune encephalomyelitis or systemic lupus erythematosus.

### BACKGROUND OF THE INVENTION

The better understanding of immunopathogenic pathways involved in the onset of autoimmune diseases, chronic inflammations or other immune-mediated diseases, have allowed research to identify the key role cytokines such as IL-6, IL-23, TNF-α, TNF-β, IL-1 β, IL-12, IL-18 and IFN-γ play in T-cell mediated autoimmune diseases. In particular, the experimental evidence suggests a key pathogenetic role for TNF-α in the pathogenesis of rheumatoid arthritis. This evidence has been successfully translated to the clinical stage as specific inhibitors, namely a neutralizing monoclonal antibody and a TNF receptor fusion protein, which are currently approved for the treatment of rheumatoid arthritis patients.

Double blind clinical studies have proven that the neutralization of TNF-α can completely abrogate the early stage of inflammation. Specific inhibitors of TNF-α can prevent the inflammation, in contrast to acetylsalicylic acid that subsides the inflammation. At first, the treatment was administered to patients with advanced disease. Upon the great beneficial success of the medication, physicians began to treat patients at an early stage of the disease. Treatment with the anti-TNF-a drug is now utilized in other autoimmune diseases, including Crohn's disease and psoriasis. However, the production of the specific TNF-inhibitors is complex and expensive. Moreover, anti-TNF inhibitors can be only given parenterally and their chronic use may involve a greater risk of developing tuberculosis.

The demonstration of the beneficial effects of specific TNF-inhibitors in rheumatoid arthritis, Crohn's disease and psoriasis has generated efforts to discover orally available small compounds that inhibit the synthesis and/or the action of endogenous TNF and possibly other cytokines that include IL-18, IL-6, IL-23, IFN- γ, IL-1, IL-12, or other cytokines.

Autoimmune diseases are immunomediated diseases that can be defined according to the organ that is attacked, the attacking mechanism, mediation of the autoantibody or T-cells, and development of the chronic inflammation processes via cytokines. Increasing evidence suggests an important pathogenic contribution of autoimmune phenomena to atherosclerosis, psychiatric disease schizophrenia, epilepsy, baldness, peptic ulcer disease, and others.

Autoimmune diseases can affect every organ or tissue. There are autoimmune diseases that affect the nervous system such as multiple sclerosis. This severe disease damages the brain and causes paralysis. Other autoimmune diseases that affect the nervous system are myasthenia gravis and Guillain-Barre syndrome. Myasthenia gravis is a disease characterized by extreme muscle weakness in which the receptor that transmits electric impulse from the nerve to the muscle is destroyed. Without the transmission, there is no muscle contraction and therefore muscle weakness develops. Guillain-Barre syndrome may develop after an infection or vaccination. There are autoimmune diseases that affect the joints, such as lupus and rheumatoid arthritis that causes deformities to the joints. Finally, there are autoimmune diseases that damage the heart, kidneys, and lungs.

Accordingly, there is a need for broad base inhibitors to suppress immune disorders including tempering increased levels of TNF-α, IL-18, IL-6, and IL-23. Additionally, there is still a need for new treatments that ameliorate, reduce and/or treat autoimmune diseases that manifest through a pro-inflammatory pathway involving increased levels of TNF-α, IL-18, IL-6, and IL-23, including arthritis, type I diabetes, colitis, uveitis, and systemic lupus erythematosus, for example.

### SUMMARY OF THE INVENTION

The subject-matter for which protection is sought is as defined in the appended claims.

The present invention provides a compound, wherein the compound is 3-phenyl-4,5-dihydro-5- isoxazoleacetic acid represented by the formula: 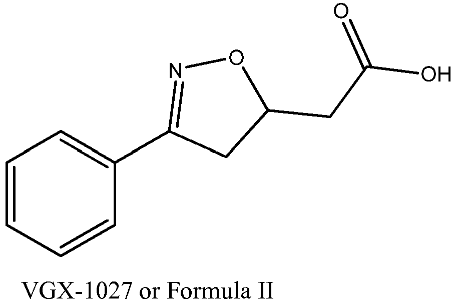 for use in a method of treating autoimmune encephalomyelitis or systemic lupus erythematosus.

The present invention also provides a pharmaceutical composition comprising the compound of the invention for use in a method of treating autoimmune encephalomyelitis or systemic lupus erythematosus.

The present disclosure is directed to a method of treating an immune disorder in a subject in need thereof. The method may comprise administering a therapeutically effective amount of a compound having the following formula: 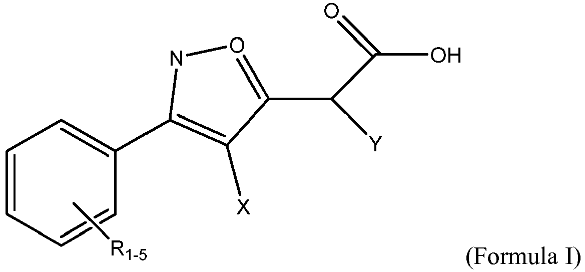 to the subject in need thereof,
wherein R₁₋₅ represents from one to five substituents independently selected from hydrogen, nitro, cyano, C₁-C₃-alkyl, halogen, carboxy, amino, trifluoromethyl, hydroxy, C₁-C₃-alkoxy groups,
X is hydrogen, halo, N₃, SH, =O, =CH₂, an aromatic, preferably phenyl, ring optionally substituted by R₁₋₅ groups as defined above, amino, mono- or disubstituted amino groups wherein the substituents are selected from C₁-C₄ alkyl, phenyl or benzyl groups optionally substituted by R1-5 groups as defined above,
Y is hydrogen, allyl C₁-C₄, amino, or a group of formula --(CH₂)₀₋₁A wherein A is an aromatic, preferably phenyl, ring optionally substituted by R₁₋₅ groups as defined above with the proviso that when X and Y are hydrogen, R₁₋₅ cannot represent a 4-hydroxy or 4-alkoxy groups.

The compound of formula I may be 3-phenyl-4,5-dihydro-5-isoxazoleacetic acid represented by the formula:

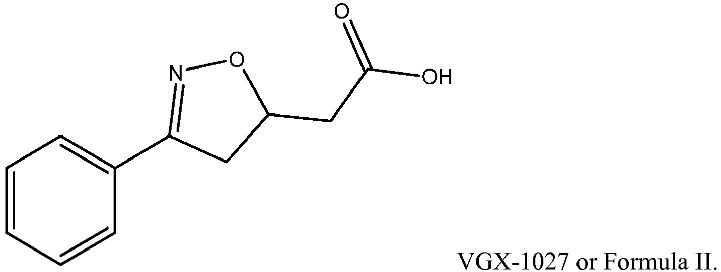

The compound of formula I may be 3-phenyl-5-isoxazoleacetic acid, represented by the formula:

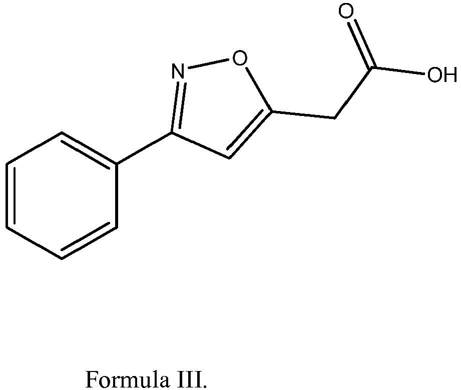

The disorder may be an autoimmune disorder, Crohn's disease, psoriasis, inflammatory dermatoses, type I diabetes, HIV infection, cancer, ischemia-reperfusion, hepatitis, and/or Guillain-Barre syndrome. Preferably, the disorder can be an autoimmune disorder. The autoimmune disorder may be rheumatoid arthritis, type II diabetes, multiple sclerosis, or acute graft rejection, for example.

The disorder may be treated or alleviated by inhibition of TNF-α production/secretion; the production of Th17 T cells; the production of IL-18; the production of IL-6; the production of IL-23; the inhibition of STAT3 activation; and a combination thereof.

The present disclosure is also directed to methods of treating a disease characterized by increased levels of IL-6, IL-23, or IL-18. These methods may comprise administering a therapeutically effective amount of a compound having formula I as shown above and described herein. The compounds of formula I may be 3-phenyl-4,5-dihydro-5-isoxazoleacetic acid. See Formula II above. The compound of Formula I may be 3-phenyl-5-isoxazoleacetic acid. See Formula III above. The disease associated with, or characterized by, increased levels of IL-6 may be Type II diabetes, plasmacytosis, rheumatoid arthritis, systemic-onset juvenile chronic arthritis, osteoporosis, psoriasis, autoimmune disease, such as antigen-induced arthritis, allergic encephalomyelitis, inflammatory bowel disease, or Castleman's disease. The disease associated with, or characterized by, increased levels of IL-18 may be coeliac disease, periodontal disease, or Crohn's disease. The disease associated with, or characterized by, increased levels of IL-23 may be one of autoimmune inflammation, such as autoimmune encephalomyelitis.

### BRIEF DESCRIPTION OF THE DRAWINGS

Figure 1 shows the plasma concentration of VGX-1027 in mice post intraperitoneal (i.p.) administration.
Figure 2 shows the effect of VGX-1027 on (A) TNF-α production following stimulation with IL-1β/INF-γ and (B) cell death following stimulation with either IL-1β/INF-γ or IL-1β/INF-γ/TNF-α. Both results were determined 48 hours post stimulation, and VGX-1027 was added at the time of stimulation.
Figure 3 shows the IL-1β and TLR pathways that are required for the production of inflammatory cytokines. See Trinchiere and Sher, 2007, Nat. Rev. Immunol., 7:179-190.

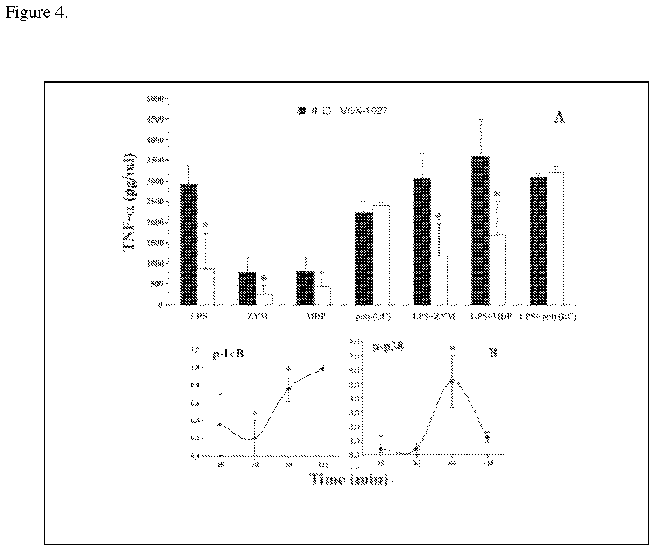

Figure 4 shows VGX-1027 inhibits the production of TNF-α following TLR-2, -4, and/or -6 activation, but not TLR-3. (A) Thioglycollate-elicited macrophages (1 x 106/ml) were stimulated with either LPS (5 µg/ml), zymosan (ZYM, 50µg/ml), MDP (25µg/ml), or Poly(I:C) (50µg/ml) in the presence or absence of VGX-1027 (10µg/ml). TNF-α was measured 48 hours post treatment. (B) Relative phosphorylations of I-κBα and p38 were measured at the indicated time points following stimulation with LPS (5 µg/ml). The γ-axis is presented as fold change in comparison to the values obtained from LPS-treated cultures alone. * p < 0.05.
Figure 5 shows VGX-1027 is non-cytotoxic and exhibits cell-type-specific inhibition of LPS-induced TNF-α production. (A) Cell viability was measured via the MTT assay after 48 hours of treatment. (B) TNF-α levels were measured 48 hours post LPS (5µg/ml) stimulation. (C) CD4 T cells were stimulated with either ConA (10µg/ml) or anti-CD3/CD28 beads (10µg/ml), and proliferation was measured via 3H-thymidine incorporation.
Figure 6 shows VGX-1027 inhibits LPS-induced cytokine production of DCs. In vitro-derived human DCs were treated with LPS (5 µg/ml), with or without VGX-1027 (10mM) for 72 hours. Concentrations of the cytokines in the media were measured via ELISA.
Figure 7 shows VGX-1027 inhibits LPS-induced maturation of dendritic cells in vivo.
   Mice were given VGX-1027 (0.5mg/mouse) or a control, and then injected with LPS (25µg/mouse) 1 hour later. At 3 hours post LPS administration, the mice were sacrificed, and (A) % of splenic DCs with CD86 and (B) MFI of CD86 from the CD86+ cells were measured.

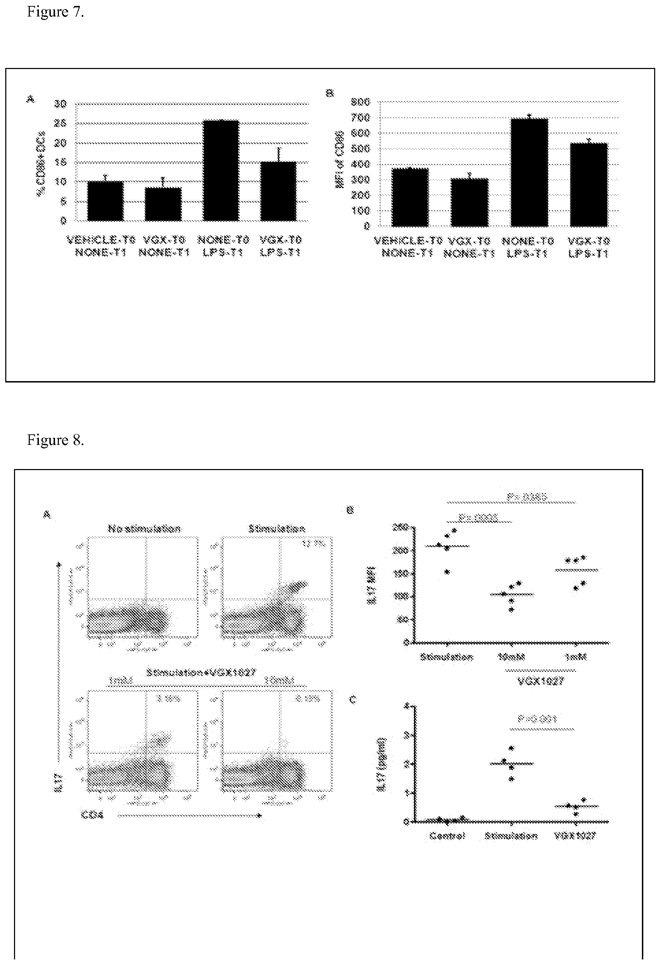

Figure 8 shows VGX-1027 prevents the development of Th17 T cells through a cell-autonomous mechanism. Purified CD4 T cells (CD3⁺ CD4⁺ CD45RA⁺ CD25⁻ HLA-DR⁻) were incubated with 10 µg/ml of IL-2, 10ng/mL of IL-1β, 10ng/ml of IL-23, 1ug/ml of anti-IL-4, 1ug/mL of anti-IFNy and anti-CD3/CD28 activation beads at a ratio of 1 bead per cell plus TGF-β (Ing/mL). On day 6, the cells were incubated in the presence of 50ng/ml of PMA and 500ng/ml of ionomycin for 5 hours. The cells were analyzed for intracellular staining of IL-17A and IFNγ. (A) FACS analysis of CD4+ and IL-17+ cells with the percentage shown in the upper right quadrant. (B) Mean Fluorescent Intensity (MFI) of IL-17+ T cells. (C) Concentration of IL-17 in the media. VGX-1027 concentration was 10mM.
Figure 9 shows that VGX-1027 protects mice from a lethal dose of LPS. VGX-1027 was administered intraperitoneally (A) or orally (p.o.) (B), and its effect on cumulative rate of lethality induced by LPS was evaluated. Data from 2 to 3 independent experiments are shown. Statistical analysis was performed by logrank (Mantel-Cox). *p<0.05 was taken as significant. Each group is compared to the vehicle-treated group.
Figure 10 shows that VGX-1027 represses CAR-induced pleurisy. VGX-1027 was administered via the intraperitoneum or orally, and the production of exudates volume (A), PCN infiltration (B), and MPO activity (C) in the lungs was determined. Data are means ± SD from 2 independent experiments.

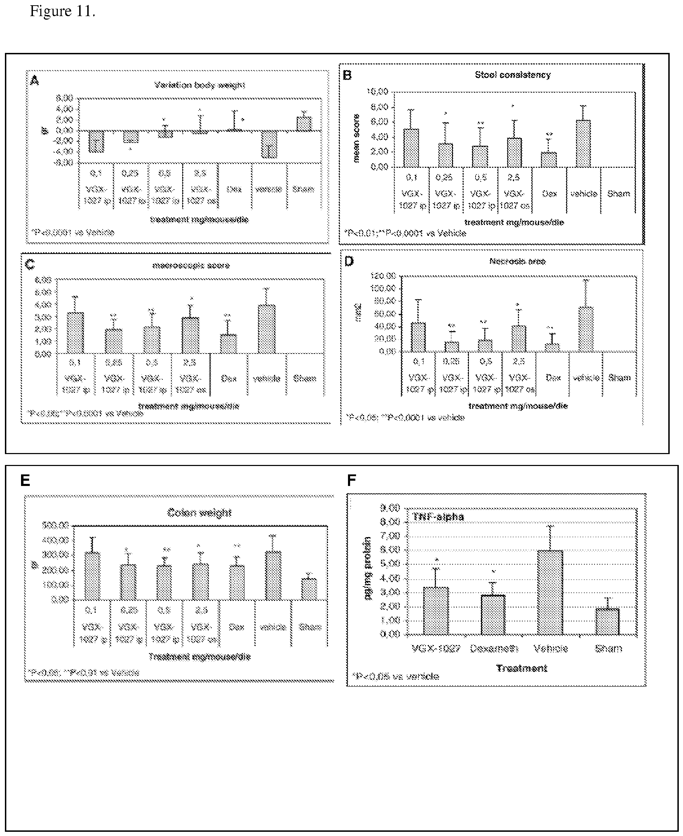

Figure 11 shows the effect of VGX-1027 on DNBS-induced colitis. Mice were injected with DNBS into the rectum and administered VGX-1027 an hour later; 4 days after, the mice were sacrificed, and the colons were isolated and analyzed. Variation in total body weight (A), stool consistency (B), macroscopic score (C), necrotic areas 9D), colon weight (E), and amount of cytokines in colon homogenate were determined. Macroscopic score was determined by the following criteria: 0 = no damage; 1 = localized hyperaemia and/or oedema; 2 = linear ulcer < half of the width of the colon; 3 = linear ulcer > half the width of the colon; 4 = circular ulcer < 1 cm; 5 = circular ulcer between 1 and 2 cm; 6 = circular ulcer > 2 cm.

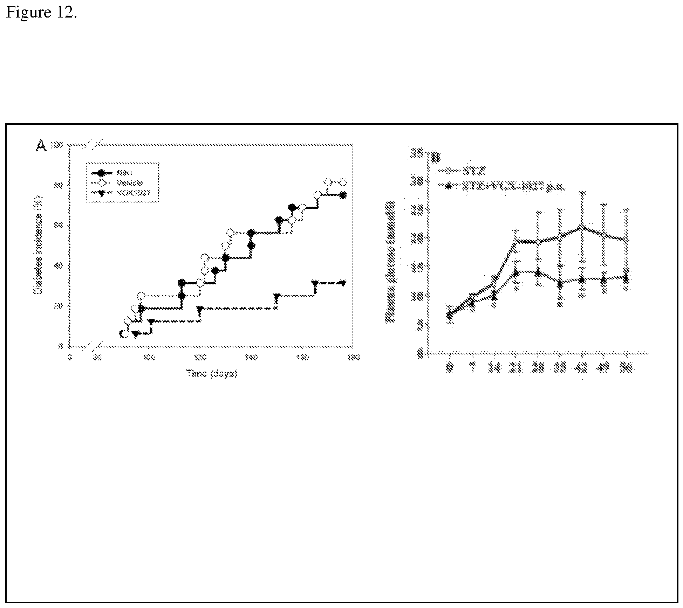

Figure 12 shows that VGX-1027 prevents the onset of diabetes in both spontaneous and STZ models. (A) Euglycaemic female 12 week old NOD mice were treated intraperitoneally with either 20 mg/kg bw VGX-1027 or its vehicle six times a week from the 12th until the 25th week of age. An additional control group of mice was left untreated. Each group consisted of 16 mice. The data are representative of two independent experiments that were merged because of interstudies variability lower than 10%. Statistical analysis was performed by Logrank (Mantel-Cox). (B) Plasma glucose levels in control CBA/H mice receiving STZ (40 mg/kg/day, for 5 consecutive days) in conjunction with 12 daily intreperitoneum injections of vehicle (STZ), or in mice treated with STZ and VGX-1027 given as a continuous 12-day treatment. 100 mg/kg bw of VGX-1027, administered orally from day -1 to day +10. Results from a representative experiment are presented as mean ± SD for seven to eight mice per group. *p<0.05 refers to treatment with MLD-STZ.
Figure 13 shows VGX -1027 counteracts the uveitis-inducing effect of LPS in rats. Rats were treated with VGX-1027 at 30 minutes (A) and 6 and 12 hours (B) after LPS injection 9200 g/footpad). The mice were evalutated 16 hours post LPS injection. Dexamethasone (1mg/kg) was administered as a positive control. *p<0.0001 vs. vehicle.
Figure 14 shows the effects of VGX-1027 therapy on the development of type II collagen-induced arthritis (CIA). VGX-1027 was administered intraperitoneally and were measured for an arthritic score (A), cumulative arthritic score (B), hind paws edema (C), and histological score (D) were measured. Data are shown as mean values ± SD form 2 independent experiments. Statistical analysis was performed by Student's t test. *p< 0.0001 was taken as significant.
Figure 15 shows VGX-1027 immune effects ex vivo in peripheral blood mononuclear cells (PBMCs). Normal human PBMCs were isolated and cultured in the presence of LPS (5µg/ml) with or without VGX-1027 treated plasma. Plasma samples were obtained by blood collection before and on the 5^{th} day after VGX-1027 dosing in steady state. Cell free supernateants were analyzed for production of TNF-α and IL-1β 24 hours post treatment. Cytokine levels (pg/ml) were assayed by ELISA. Representative results were correlated with pro-inflammatory cytokine inhibition.

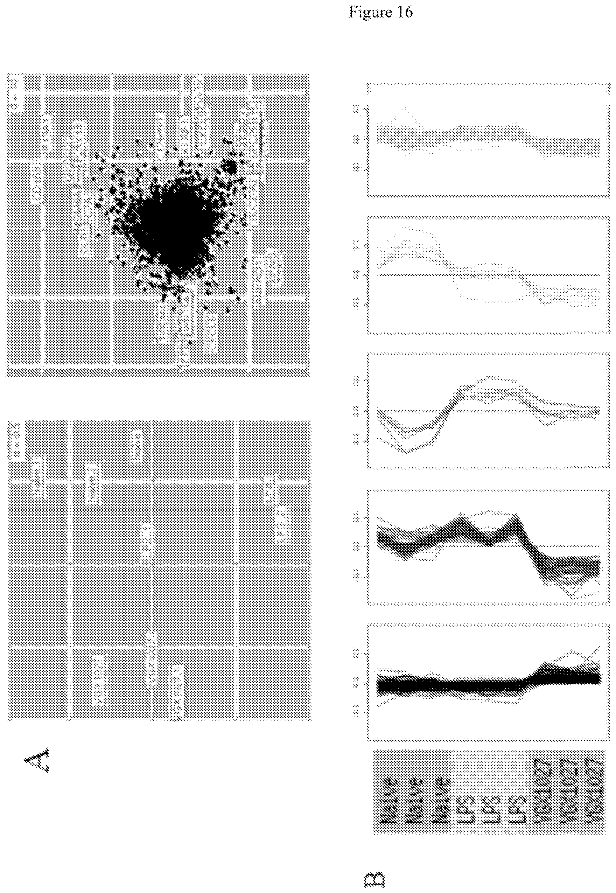

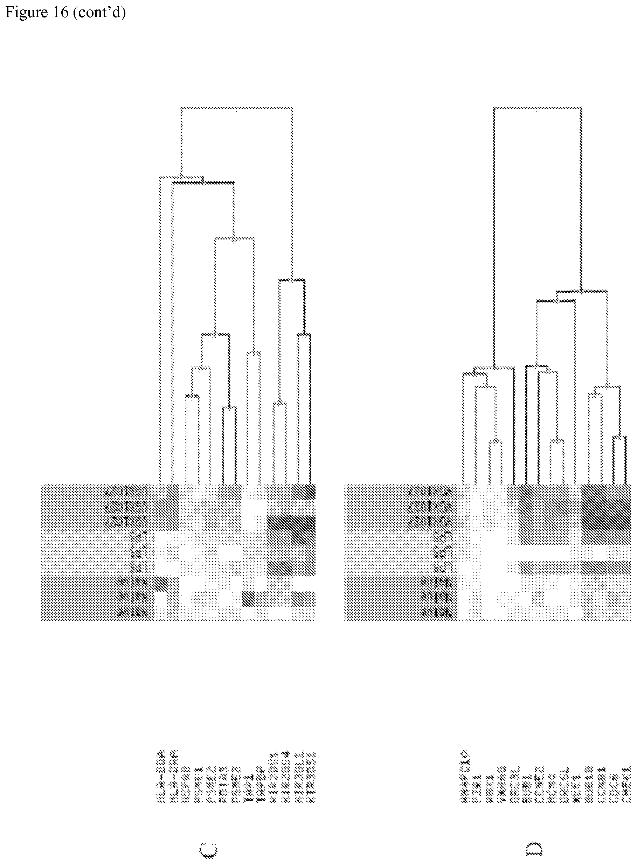

Figure 16. (A) Principal Component Analysis (PCA) of the transcriptome expressed on either naive PBMCs, PBMCs stimulated in vitro with LPS or treated with VGX1027 following LPS administration. PCA was carried on all genes to determine expression trends within the data set. (B) K-mean clustering of differentially expressed genes. The x-axis corresponds to the experimental conditions, the y-axis to the expression levels. Each line represents a gene. (C, D) Differential regulation of genes related to the KEGG categories "Antigen Processing and Presentation" (C) or "Cell Cycle" (D) is shown as heatmap after unsupervised hierarchical clustering.
Figure 17. Western blot (A) and relative densitometric analysis (B) of the levels of phospho-STAT3 in CD4+ T cells after stimulation with IL-6.
Figure 18. Survival proportion of mice challenged with SEB and either treated with a single i.p. dose of VGX-1027 or vehicle. Survival was monitored over 48 hours.
Figure 19. Effects of VGX-1027 administration on survival rate (A), proteinuria (B) and serum anti-dsDNA antibodies (C) of NZB/NZW F1 mice treated for 20 weeks with either VGX-1027 or vehicle.
Figure 20. Histopathological analysis (A) and relative phospho-STAT3 (B) levels in kidney sample of NZB/NZW F1 mice treated for 20 weeks with either VGX-1027 or vehicle.

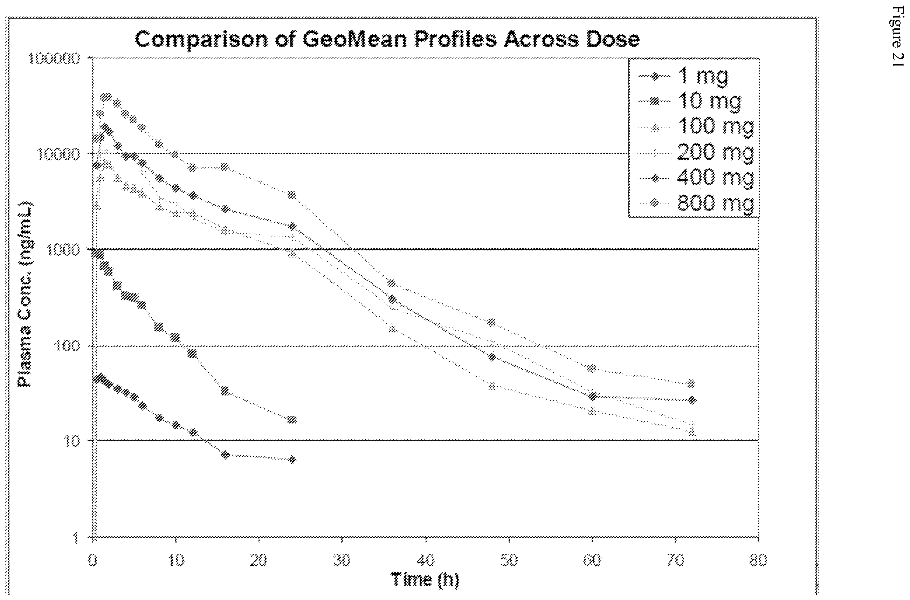

Figure 21. Displayed is a line graph showing the PK levels in human subjects that were part of a placebo-controlled, ascending single-dose study to evaluate the safety and pharmacokinetics of VGX1027 in healthy subjects. Subjects were administered from 1 mg to 800mg of VGX1027.
Figure 22. Displayed is a line graph showing the PK levels in human subjects that were part of a placebo-controlled, ascending multiple-dose study to evaluate the safety and pharmacokinetics of VGX1027 in healthy subjects. Subjects were administered from 1 mg to 400mg of VGX1027.

### DETAILED DESCRIPTION OF THE INVENTION

Isoxazoline compounds have been disclosed as inhibitors of phosphodiesterase type IV (U.S. Pat. No. 5,716,967), fibrinogen receptor antagonists (U.S. Pat. No. 5,849,736), inhibitors of TNF release (U.S. Pat. No. 6,114,367) and as macrophage migration inhibitory factor (MIF) antagonists (WO 02/100332).

It has now been found that compounds of formula I, differing from those disclosed in U.S. Pat. No. 5,716,967, U.S. Pat. No. 5,849,736, and WO 02/100332 in the absence of an hydroxyl or alkoxy group in the para position of the phenyl ring, inhibits IL-1β, TNF-α and IL-10 synthesis, and prevents NFkB activation. 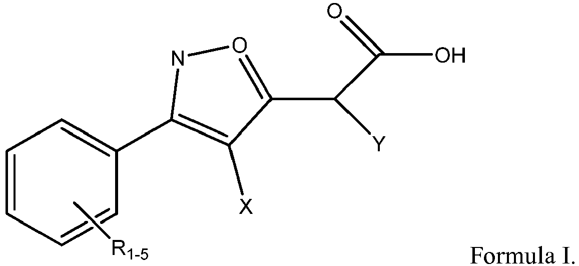 In formula I, R₁₋₅ represents from one to five substituents independently selected from hydrogen, nitro, cyano, C₁-C₃-alkyl, halogen, carboxy, amino, trifluoromethyl, hydroxy, C₁-C₃-alkoxy groups, X is hydrogen, halo, N₃, SH, =O, =CH₂, an aromatic, preferably phenyl, ring optionally substituted by R₁₋₅ groups as defined above, amino, mono- or disubstituted amino groups wherein the substituents are selected from C₁-C₄ alkyl, phenyl or benzyl groups optionally substituted by R₁₋₅ groups as defined above Y is hydrogen, allyl C₁-C₄, amino, or a group of formula --(CH₂)₀₋₁A wherein A is an aromatic, preferably phenyl, ring optionally substituted by R₁₋₅ groups as defined above with the proviso that when X and Y are hydrogen, R₁₋₅ cannot represent a 4-hydroxy or 4-alkoxy groups.

Some of the compounds of formula I are known or can be prepared by known methods. The compound wherein R and R' are both hydrogen and a 4,5-dihydro isoxazoline ring system have been reported by Synth. Comm., 1998, 28(13), 2457-2466 and from Synth. Comm., 1997, 27(16), 2733-2742.

The disclosure described in this document concerns therefore the use of compounds of formula I for the preparation of a medicament for the treatment of those diseases which may be alleviated by the inhibition of TNF-α, TNF-β, IL-1 β and/or IL-10 associated or not to dysregulated activation of NFkB. Preferred compounds of formula I are 3-phenyl-4,5-dihydro-5-isoxazoleacetic acid (hereinafter referred to as VGX-1027 or Formula II): 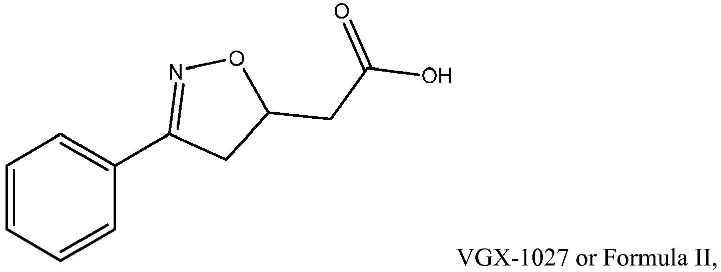 and 3-phenyl-5-isoxazoleacetic acid (hereinafter Formula III): 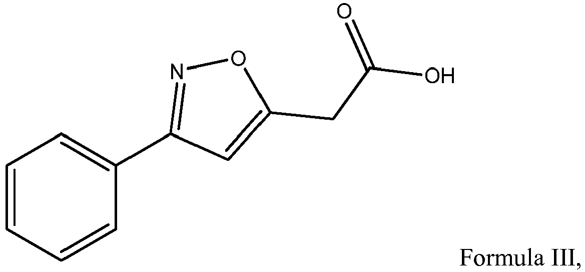 as well as their pharmaceutically acceptable salts, such as sodium, potassium, calcium salts and the like. VGX-1027 is particularly preferred.

The compound of the invention is particularly useful for the treatment of human pathologies mediated by TNF-α, TNF-β, IL-1β and/or IL-10 associated or not to an activation of NFkB. Said pathologies include immuno-inflammatory, autoimmune and infectious diseases including rheumatoid arthritis, Crohn's disease, psoriasis and inflammatory dermatoses, type I diabetes, HIV infections, cancer, ischemia-reperfusion, hepatitis, multiple sclerosis, Guillain-Barre syndrome and prevention of acute graft rejection.

The invention therefore also concerns pharmaceutical compositions comprising a compound of the invention as the active ingredient in admixture with a suitable carrier or excipients.

### a. Preparation of VGX-1027

Benzaldehyde (32.8 mmol) and hydroxylamine hydrochloride (33 mmol) were dissolved in methanol (100 ml) followed by addition of sodium carbonate (65 mmol). Overnight reaction gave the oxime derivative in 95% (2, 30.4 mmol). Chlorination of the oxime using N-chlorosuccinimide (31.6 mmol) in DMF (100 ml) quantitatively furnished chlorooxime (3). Compound 3 was then dissolved in THF/H2O (*0/20) and treated with 3-butenoate (24.5 mmol) and sodium carbonate (73.6 mmol). After completion, (12 h), the product was extracted with ethyl acetate and the organic extracts were washed with brine and dried over magnesium sulfate. GIT-27 was crystallized from ethyl acetate/heptane mixture. The structure was confirmed by 1H-NMR, 13C-NMR and mass spectroscopy. The daily dosage regimen will presumably vary within wide ranges, for instance from 0.1 to 10 mg/kg body weight.

### b. Absorption, Distribution, Metabolism, and Elimination (ADME) of VGX-1027

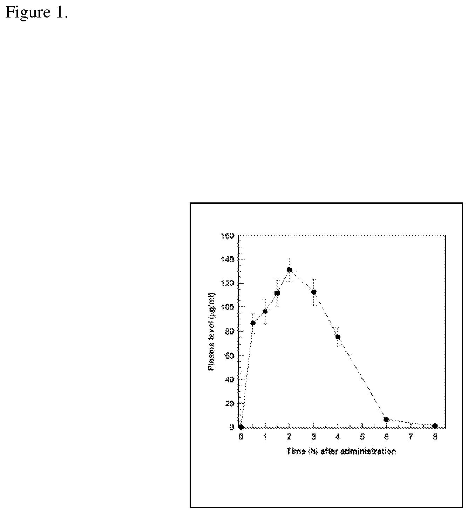

To determine the pharmacokinetic properties of VGX-1027, drug concentrations were analyzed in the plasma of mice. The drug was measured post intraperitoneum administration at 20 mg/kg birth weight in 500 mM Na₂HPO₄, which is a therapeutically active concentration. The peak plasma concentration of the drug was 130 µg/ml (Cmax) and was observed after 2 hours (Tₘₐₓ) post application. See Figure 1. The drug concentration was greater than 80 µg/ml, which is well above the concentration required to exert immunopharmacological effects, by 30 minutes post injection, and eventually decreased below 10 µg/ml after 6 hours post injection. Based on the linear part of the pharmacokinetic profile, the t_{1/2} of VGX-1027 is approximately 90 minutes.

With respect to the oral bioavailability in mice and dogs, VGX-1027 was rapidly absorbed. The highest plasma concentrations generally occurred in 1-2 hours. There appears to be a low potential for drug-drug interactions, as there was no significant inhibition by CYP450 enzymes (1A2, 2C9, 2C19, 2D6, or 3A4). The highest level of inhibition was ∼35% for 2C19 at a 30µM concentration. Metabolic disruption of the isoxazole ring, single oxidation and single glucuronidation are not pathways of metabolism as no metabolite peaks were observed for the 3 monitored masses for any species. Lastly, VGX-1027 is stable in human liver S9 fractions (with phase 1 and 2 cofactors) with t_{1/2} > 90 minutes, which indicates a low likelihood of metabolism in the liver. There was no significant inhibition of COX-1 or COX-2 activity.

VGX-1027 was negative in a radioligand binding assay (MDS Pharma HitProfiling Screen) that tested 30 primary molecular targets at concentrations up to 10 µM. No significant response (≥50% inhibition or stimulation for Biochemical assays) were noted.

### c. Toxicology Studies in Dogs and Mice

Twenty-eight day toxicology studies were performed in both mice and dogs with increasing concentration to achieve toxicity. The following are the results of those studies.

At the highest dose (500 mg/kg/day) in mice, notable changes found in kidneys, liver, spleen, testis, epidydimus and bladder, including increased organ weights and changes in histopathology.

At the highest dose [100mg/kg/day] in dogs, 3 early deaths - 2 in moribund condition, 1 due to infection who had a swollen forelimb due to a pus filled pocket that was positive for *Staphylococcus aureus.*

In a rising MTD study in mice, early deaths were noted in 1000, 1500 and 2000 mg/kg/day; observations included tremors, mixed convulsions, ptosis, decreased activity, cold to the touch and/or impaired hind limb function. (Approximate Cmax @ [1000 mg/kg/day] was 400 mg/mL). Based on these studies, 10 mg was determined to be a safe starting dose in the human study.

In summary, intraperitoneum administration of VGX-1027 (20 mg/kg body weight) leads to a Cmax of 130 µg/ml and a Tmax of approximately 2 hours in mice. VGX-1027 is not subjected to extensive biotransformation in mice and dogs. There is low potential for drug-drug interactions. At therapeutic doses, VGX-1027 did not exhibit toxicity. Further, VGX-1027, when given as an oral pill, was well tolerated in humans. No dosing toxicities were observed for up to 800 mg QD single dose and for up to 200 mg bid for 5 days. No Grade 3 or 4 AEs were reported, and no SAEs were reported. CAT002 urine PK analysis showed high drug levels in urine.

### 2. VGX-1027 Effects on Specific Conditions and in Model Systems

The pharmacological activity of VGX-1027 has been extensively studied both *in vitro* and *in vivo.* More particularly, experiments under the following conditions and models have been carried out, according to known and well-established methods: lymphoproliferation, TNF-α, Interferon (IFN)- γ, IL-1 β, IL-10 and MIF synthesis/secretion; NF-kB activation in human macrophages; murine LPS-induced lethality; murine Carrageenan-induced pleurisy; murine type II collagen-induced arthritis; murine oxazolone-induced hypersensitivity; murine immunoinflammatory diabetes induced by multiple low doses of streptozotocin; and chemically-induced colitis in mice. In addition, further studies related to VGX-1027 were conducted to ascertain its effect on immune-mediated diseases such as autoimmune disease and chronic inflammation; on in vitro inflammation and Th17 T cell development; on the activation of monocytes and macrophages; and on the development of Th17 T cells via cell-autonomous and non-cell-autonomous mechanisms. Still further, VGX-1027 was orally administered to various mice models to study its ability to effectively treat autoimmune disease. The following mouse models of various autoimmune diseases were investigated: LPS-induced lethality (endotoxin-induced); Pleurisy (lung inflammation); chemical induced immunoinlammatory colitis (DNBS model); STZ-induced diabetes in NOD mice (type I diabetes); type II collagen-induced arthritis (rheumatoid arthritis); endotoxin-induced uveitis (EIU) in male Lewis rats (model of inflammatory ocular disease); and the staphilococcus aureus enterotoxin B (SEB)-induced shock and the NZB/NZW F1 model of systemic lupus erythematosus (SLE). The results obtained performing the experiments listed above are summarized below.

### a. In Vitro Effect of VGX-1027 on Proliferation of Lymphocytes and on TNF, IFN- γ, IL-10 and MIF Synthesis and Secretion by Macrophage and Lymphocyte

Peritoneal macrophages (PM) and splenic mononuclear cells (SMNC) were isolated from normal mice, cultured in nutrient medium and stimulated with LPS and/or ConA in the absence or presence of VGX-1027. The results clearly show that VGX-1027 markedly inhibited the production of TNF-α by both PM and SMNC and inhibited the production of IL-1 β IL-10 by PM. VGX-1027 had no effect on MIF expression in either cell type, on proliferation of SMNC, and on IL-10 and Interferon (IFN) γ production by SMNC.

Proliferative and cytokine synthesis capacity of lymphocytes and macrophages isolated from G127 treated mice.

These experiments were carried out to explore if intraperitoneal (i.p.) or oral administration (p.o.) in mice with VGX-1027 could influence the proliferative response of SMNC and the production of IL-1β, IL-10, TNF-α, IFN-γ and MIF from isolated PM and SMNC. PM and SMNC isolated from mice treated in vivo with VGX-1027 for 7 consecutive days intraperitoneally (0.5 mg/mouse), or orally (2.5 mg/mouse), or isolated from control animals that received VGX-1027 vehicle (Na₂HPO₄/H₂O), or were without any treatment. 24 h after the last VGX-1027 treatment, PM and SMNC were collected for further in vitro stimulation and analysis.

This *ex vivo* experiment clearly shows that PM's isolated from VGX-1027 treated mice exhibit impaired synthesis of IL-1 β, TNF-α and IL-10 while showing a normal pattern of MIF expression upon mitogenic stimulation. On the other hand, the SMNC isolated from VGX-1027 treated mice show a normal capacity to proliferate, a normal capacity to produce TNF-α, IL-1 β, IL-10, and IFN- γ upon stimulation, and a normal expression pattern of MIF expression.

The findings from "in vitro" and "ex vivo" experiments concordantly show that VGX-1027 down-regulates the production of IL-1 β, TNF-α and IL-10 by PM without altering the capacity of SMNC to produce IFN-γ and does not affect the expression of MIF by PM and SMNC. In contrast, the *"in vitro"* experiments show that VGX-1027 reduced TNF-α production by SMNC while the *"ex vivo"* experiments do not support this evidence.

### b. Effect of VGX-1027 on NF-kB Activation in Human Macrophages

The results from this *in vitro* test indicate that VGX-1027 powerfully inhibits in a dose-dependent fashion the DNA binding of NF-kB that LPS induced in monocyte-derived human macrophages. These results provide evidence for the mechanism of action of VGX-1027 and suggest that the inhibitory effects that VGX-1027 exerts on macrophage synthesis of IL-1 β, TNF-α and IL-10 might secondary to the inhibition of NF-kB binding to DNA.

### c. Effect of VGX-1027 on LPS-Induced Lethality In Vivo

This *in vivo* study showed that the therapeutic administration of VGX-1027 powerfully counteracts LPS-induced lethality in mice.

As expected, most of the control mice either untreated or that received the vehicle of VGX-1027 succumbed to LPS-challenge within 3 days. After this period of observation, one-week follow-up of the surviving mice revealed a full clinical recovery. In contrast, the treatment with VGX-1027 given either orally or intraperitoneally administration protect the mice from the lethal effects of LPS in a dose-dependent fashion. When given orally, VGX-1027 was effective at the dose of 2.5 mg/ mouse and dosed out both at 1.5 and 3 mg/mouse. When given intraperitoneally, VGX-1027 exhibited a wider therapeutic window with dose-dependent protection against LPS-induced lethality between 0.25 and 0.5 mg/mouse. VGX-1027 was also capable of significantly reducing LPS-induced lethality when it was given both orally and intraperitoneally at similar doses under a prophylactic regime (e.g. -24 and -1 hour prior to LPS).

### d. Effect of VGX-1027 on Oxazolone-Induced Dermatitis

Oxazolone-induced hypersensitivity is an immune-mediated dermatitis that can be induced by two epicutaneous exposure to oxazolone. While the first application of oxazolone sensitizes the mice, the second elicits the hypersensitivity reaction. The dermatitis manifestations appear 18 hours after the second epicutaneous exposure to oxazolone, both ear thickness and weight markedly and progressively increase. The *in vivo* studies were planned to assess the pharmacological activity of VGX-1027. The mice were treated during or before the sensitization phase. This experiment shows that ear thickness and weight markedly, and progressively increased in the mice that had been treated with either the vehicle of VGX-1027 or were left untreated. In contrast, the inflammatory swelling of the ear, as measured by the increment of ear thickness and weight, was significantly suppressed by VGX-1027. In similar conditions, dexamethasone reduced the thickness, but not the weight of the ears.

To evaluate the effects of VGX-1027 on the elicitation phase of oxazolone-induced dermatitis, VGX-1027 treatment was given one hour after the second epicutancous exposure to oxazolone and the beginning of the immunoinflammatory responses. Under this experimental regime, VGX-1027 only partially affected the development of allergic dermatitis. In comparison with control animals, the mice, treated with VGX-1027 only exhibited a significant decrease in the thickness, but not in weight of the ears. In this experimental regime, dexamethasone did not affect the increase of ear thickness and weight.

The result of this experiment shows that treatment with VGX-1027 can successfully suppress the dermatitis induced by repeated epicutaneous exposure to oxazolone when VGX-1027 was administered upon profilactic regime, but prior to the sensitisation phase of the disease.

Since dexamethasone is a powerful immunosuppressant widely and effectively used for the topical treatment of human inflammatory skin diseases, VGX-1027 shows pharmacological potentials for the systemic or topical use in the treatment of immune-mediated or type 1 cytokine dependent skin diseases such as psoriasis, some forms of pemphigus vulgaris and cutaneous vasculitis during graft versus host disease.

### e. Effect of VGX-1027 on Streptozotocin Induced Diabetes

Both intraperitoneal and oral treatment with VGX-1027 suppressed clinical development of diabetes. Clinical development of diabetes was attempted to be induced in CBA/H mice by multiple low doses of streptozotocin. CBA/H mice developed sustained hyperglycemia over a 2-week period following MLD-STZ injections. As expected, CBA/H control mice treated with the vehicle of VGX-1027 either orally or intraperitoneally developed diabetes with sustained hyperglycemia developed over a 2-week period following MLD-STZ injections. In contrast, the diabetogenic effect of MLD-STZ was significantly reduced when the mice were treated with VGX-1027 intraperitoneally. The drug was also capable of significantly reducing development of hyperglycemia when administered orally.

The prophylactic treatment with VGX-1027 given either intraperitoneally and orally, successfully counteracted the development of hyperglycemia that is secondary to the cell-mediated immune destruction of the β cells after consecutive injections of STZ. The drug was effective using both intraperitoneal- and oral-type administrations. These results suggest that VGX-1027 possesses pharmacological properties worthy of being further considered for clinical use in the early treatment of human type 1 diabetes (T1D) as well as in the prevention of the disease in individuals at risk for its development such as those first degree relatives of patients with T1D that exhibit metabolic (defective insulin secretion) and immunologic parameters (anti- β cell autoantibodies, HLA haplotypes) associated with high risk of T1D development.

### f. Effect of VGX-1027 on DN-Induced Colitis

Within 5 days after challenge with dinitrobenzene sulfonic acid (DNB), mice develop colitis. The disease severity is evaluated based on a macroscopic score. The vehicle-treated control mice and untreated mice developed colitis with a predictable and progressive increase in inflammation and body weight loss. The progression of inflammatory colitis was significantly attenuated in those animals, which had been treated with VGX-1027 (p<0.001). In comparison with the control groups, the VGX-1027 treated animals show a significant reduction in weight loss. In agreement with this data, the increase in colon weight that accompanies the development of DNB-induced colitis was significantly lower in the mice treated with VGX-1027 as compared to the controls.

The colons of the control mice and of the VGX-1027 treated mice were removed, sectioned, fixed, and stained with hematoxylin and eosin. A reduction in the inflammatory cellular infiltrate, mucosal and muscle damage, as well as wall thickening were observed in the animals that were treated with VGX-1027 before DNB-induced colitis. The histology clearly indicates that the tissue damage is significantly reduced in the mice treated with VGX-1027 as compared to the control mice. These data confirm the anti-inflammatory efficacy of VGX-1027 in colitis. The tissue myeloperoxidase activity is a well established marker to assess the inflammatory cell (mainly neutrophils) infiltration. The myeloperoxidase activity measured in the colon tissues collected from VGX-1027 treated mice and from control mice clearly indicate that the mice treated with VGX-1027 show a significant reduction of inflammatory cell infiltration 5 days after the induction of colitis as compared to controls (p<0.0001). This result parallels the reduction of neutrophils infiltrating the same histology samples. We counted an average of 8.9 ± 1.2 (means ± SD) cells/high power field of DNB-induced animals, whereas there were only 3.37 ± 0.8 cells/high-power field in DNB-induced animals pretreated with VGX-1027 (p<0.0001). These data prove that the treatment with VGX-1027 powerfully protect the test animals from the colitis that is induced by the administration of DNB. The present data indicate that this drug hold promises for its possible use in the treatment of human IBD.

### g. Effect of VGX-1027 on Immune-Mediated Diseases Such as Autoimmune Diseases and Chronic Inflammations

The *in vitro* and *in vivo* results obtained using VGX-1027 demonstrate that this molecule has the pharmacological potential to be an interesting drug for the treatment of those diseases that are mediated by immune mechanisms such as autoimmune diseases and chronic inflammations, and some skin diseases and endocrinediseases. In general VGX-1027 has a potential in the therapy of those diseases that are mediated by IL-1 β, TNF-α or other cytokines.

Because IL-10 has anti-inflammatory properties (World J. Gastroenterol., 10; 620, 2004), the inhibitory effect that VGX-1027 exert on the macrophage synthesis of this cytokine may appear to lack consistency with the use of this drug in immunoinflammatory diseases. Nonetheless, IL-10 has also been shown to exert proinflammatory effects in different rodent models such as endotoxin induced uveitis (J. Immunol., 1995, 155: 4090-4), type 1 diabetes (J. Immunol. 2000, 165:2841-9), systemic lupus erythematosus (Arthritis Rheum. 2000, 43:1790-800.) and experimental autoimmune orchitis (Cytokine. 2003, 22:50-3.). It is therefore possible that under certain circumstances, the reduction of IL-10 synthesis may actually enhance the anti-inflammatory efficacy of a drug. This is also consistent with the fact that Cyclosporin A, which is widely used in the clinical setting for the treatment of autoimmune diseases, also down-regulates IL-10 synthesis *in vitro* (J. Exp Med. 199317:551-5). In addition, since exuberant production of IL-10 has been thought to play a pathogenic role in certain cancers (Immunol Rev. 2004 December; 202:223-36.; Br J Haematol. 2003, 122:927-33) and HIV infections (Blood. 2003, 101:2514-20.), the capacity of VGX-1027 to down-regulate the synthesis of this cytokine represents a potential rationale for the potential investigation of this drug in these diseases.

For the considered therapeutic uses, the compounds of formula I will be administered by the oral, parenteral, transdermal or transmucosal routes in form of suitable compositions in admixture with conventional carrier or excipients, prepared according to known methods. The dosage will depend on several factors, such as the seriousness of the pathology, the kind of patient (age, sex and weight) and will be anyhow easily determined by the skilled practitioner on the basis of the toxicological and pharmacokinetic properties of the drug.

### h. VGX-1027 Exhibits Anti-Inflammatory Effects in vitro and Represses Th17 T Cell Development

Insulinoma cell lines, which die following stimulation with either IL-1β/INF-γ or TNF-α/INF-γ, were used in this study. Specifically, cell lines RIN-m5F and MIN6 were treated with either IL-1β/INF-γ alone or IL-1β/INF-γ/VGX-102, and TNF-α production, which is a consequence of stimulation with these factors, was measured.

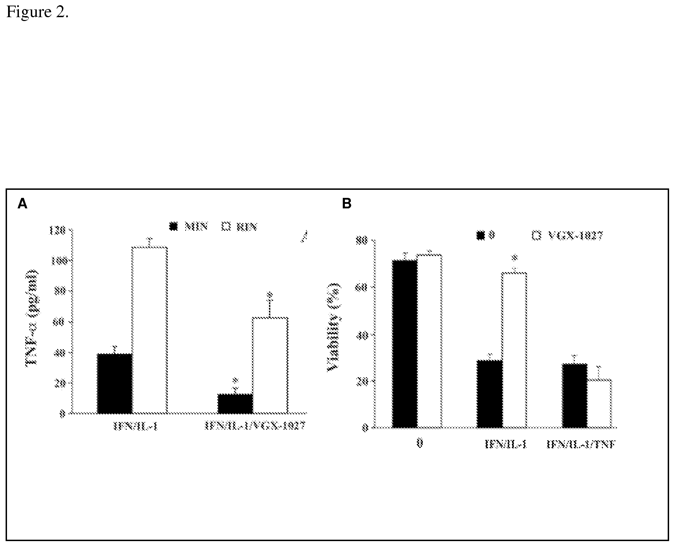

As shown in Figure 2a, VGX-1027 treatment significantly repressed TNF-α production following stimulation with IL-1β/INF-γ in both cell types. In addition to the production of TNF-α, cell death ensues following treatment of these cells with IL-1p/INF-γ. As shown in Figure 2b, only about 30% of the IL-1β/INF-γ treated cells remained viable after 48 hours of treatment; however, the co-treatment with VGX-1027 recovered cell viability to over 60%, which is similar that of the control group. More interestingly, exogenous addition of rTNF-α prevented the protective effect of VGX-1027 co-treatment, likely the result not due to INF-γ inhibition (Figure 2b). Therefore, VGX-1027 inhibits IL-1β-induction, but not TNF-α-induced, cell death.

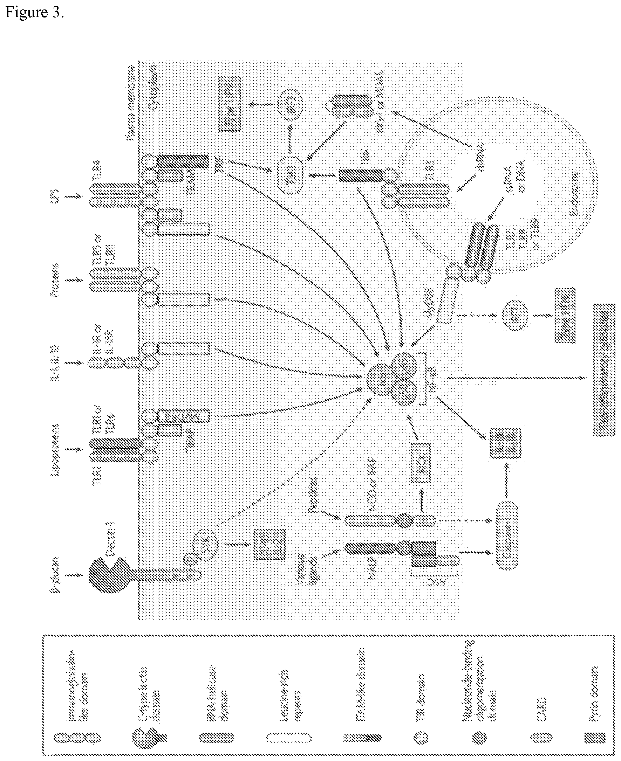

Since VGX-1027 specifically inhibits IL-1β-induction, but not TNF-α -induced cell death, VGX-1027 can target a protein downstream of the IL-1β receptor (IL-1R), but not the TNF-α receptor (TNFR). This is unlike signals that result from TNFR activation, which require signals from both IL-1R and Toll Like Receptor (TLR) to activate the MyD88-TRAF6-IRAK pathway (Figure 3) (Reviewed in Trinchieri et al., Nat. Rev. Immunol., (8) 179-190 (2007).

Thioglycollate-elicited macrophages were treated with multiple TLR agonists for 48 hours. TNF-α production was measured. The following agonists were used to activate the various TLRs. See the following Table 1.

**Table 1**

| **Agonist** | **TLR Activated** | **MyD88 Dependence** |
|---|---|---|
| LPS | TLR4 | Yes |
| Zymosan | TLR2/6 | Yes |
| Muramyl Dipeptide (MDP) | TLR2/4 | Yes |
| Poly (I:C) | TLR3 | No |

As shown in Figure 4a, VGX-1027 inhibited TNF-α production from cells that were stimulated with LPS, ZYM, and MDP, which activate either TLRs 2, 4, and/or 6. However, VGX-1027 did not affect poly(I:C)-induced TNF-α production, suggesting that TLR-3-driven TNF-α production is not inhibited by VGX-1027. A distinction between TRL-3 and the other TLRs and IL-1βR is that TLR-3 activation does not require MyD88/TRAF6/IRAKs for stimulating NF-kB activation or secretion of inflammatory cytokines. See Figure 3. (Jiang et al., J. Biol. Chem., (278) 16713-16719 (2003). In addition, it was observed that VGX-1027 inhibited LPS-induced phosphorylation of p38 and 1-kBα, indicating VGX-1027 inhibits a protein upstream of both the p38 and NF-κB, signaling pathways (Figure 4b).

As this data suggests, VGX-1027 can inhibit TNF-α secretion induced by the activation of most TLRs and IL-1R, and this effect correlates with MyD88-TRAF6-IRAK dependence and inhibition of NF-κB and p38 pathways.

### i. VGX-1027 Inhibits Activation of Monocytes and Macrophages

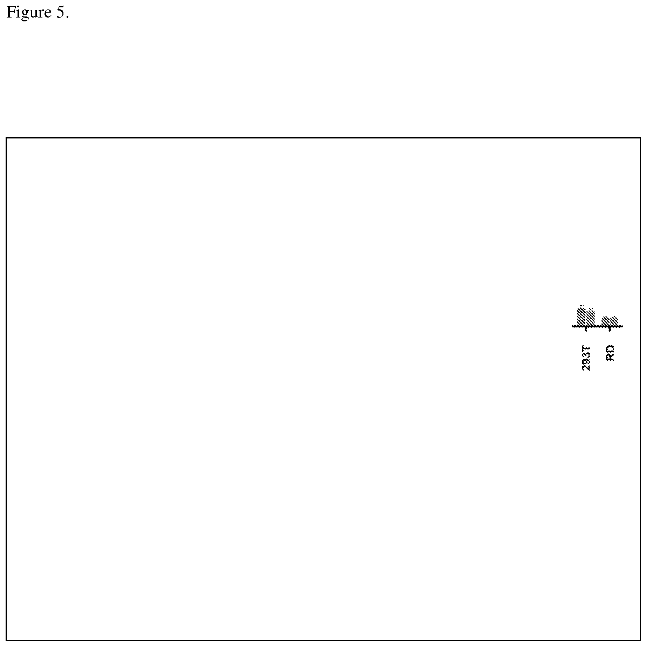

VGX-1027 was tested for whether its effects were cell-type-specific. As shown in Figure 5a, VGX-1027 treatment of various asynchronously growing cell lines did not cause toxicity as measured by the MTT assay (Figure 5a) and Pl/Annexin V staining. Greater than 80% viability was observed with 100 mM of the drug, indicating that VGX1027 by itself is not cytotoxic at even high concentrations (Figure 5a).

Next, the production of TNF-α was measured following LPS stimulation in these different cell types. As shown in Figure 5b, VGX-1027 treatment inhibited LPS-induced TNF-α production in U937s, RAWs, primary monocytes and PBMCs. Conversely, VGX-1027 treatment did not affect TNF-α production in Jurkats, undifferentiated CD4 T cells, 293Ts, or RD cells. This suggests that VGX-1027's effect is specific to monocytes/macrophages. VGX-1027-induced effects were not observed.

Since LPS treatment does not affect T cell proliferation (Imanishi et al., J. Immunol., (178) 6715-1719 (2007)), the effect of VGX-1027 on the proliferation of undifferentiated CD4 T cells was measured following canavalin A (ConA)- and CD3/CD28-induced proliferation. Canavalin A (ConA) and CD3/CD28-induced proliferation do not require MyD88-TRAF6-IRAK pathway. Consistently, it was observed that VGX-1027 affected neither the proliferation of undifferentiated CD4 T cells following either ConA or CD3/CD28 stimulation (Figure 5c), nor INF-γ production following ConA stimulation.

### j. VGX-1027 Prevents the Development of Th17 T Cells through both Cell-Autonomous and Non-Cell-Autonomous Mechanisms

Th17 T cells are critical for the development of a number of autoimmune disorders, which include EAE and CIA (See Table 2). Therefore, VGX-1027 was analyzed for whether it could inhibit the production and/or activity of either direct or indirect factors involved in the Th17 T cell development. Th17 T cell development requires TGF-β, IL-1β, IL-6, and either IL-21 or IL-23 (Manel et al., Nat. Immunol., (9) 641-649 (2008)). Moreover, the *in vivo* importance of these cytokines has been demonstrated using mouse genetics. For example, mice deficient in either IL-6 or IL-23 fails to induce Th17 T cells in a mouse experimental autoimmune uveitis (EAU) (Yoshimura et al., Rheumatology, (48) 347-354 (2009)).

**Table 2**

| **Knockout** | **Phenotype** |
|---|---|
| *p35-*/*- (IL-12)* | Susceptible to Experimental Autoimmune Encephalomyelitis ("EAE"), Collagen-Induced Arthritis ("CIA") |
| *p19-*/*- (IL-23)* | Resistant to EAE, CIA, CD40-induced colitis |
| *p40-*/*- (IL-12 and IL-23)* | Resistant to CIA |
| *INF-γ* -/-, *INF-γR*-/- | Hyper-susceptible to CIA, EAE |
| *TNF-α* -/- | Susceptible to EAE |
| *IL1R-*/*-* | Resistant to EAE |

The source of these cytokines is, at least in part, dendritic cells (DCs), which also capture antigen for presentation to T cells. In addition, a large number of studies suggest an essential role for DCs in the development of autoimmune diseases. For example, DCs are found in high numbers in the sera and synovial fluids of patients afflicted with Rheumatoid Arthritis (RA) (Zvaifler et al., J. Clin. Invest., (76) 789-800 (1985)). They are a major source of TNF-α production in psoriasis (Sabat et al., Exp. Dermatol., (16) 779-798 (2007)). In addition, they are sufficient to induce autoimmunity by presenting self antigens in a number of mouse models (Banchereau et al., Immunity, (20) 539-550 (2004)). DCs express TLRs and can be stimulated with TLR ligands to induce autoimmunity in a variety of mouse models. For example, DCs present endogenous myelin antigens to naive T cells, and as a result, polarized the T cells to the Th17 phenotype via enhanced IL-1β and IL-6 production (Miller et al., Ann. N.Y. Acad. Sci. (1103) 179-191 (2007)). In addition, activation of TLRs on DCs promotes IL-23 production, leading to the development of Th17 T cells (Van Beelen et al., Immunity, (27) 660-669 (2007)). Therefore, we first investigated if VGX-1027 could inhibit the production of IL-23 and IL-6, two essential cytokines for Th17 T cell development, from LPS-stimulated DCs.

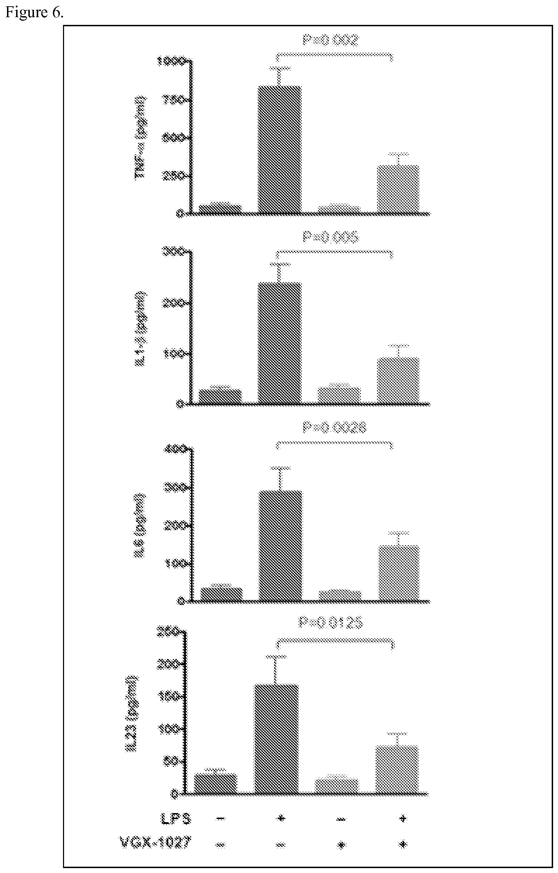

As shown in Figure 6, LPS treatment of DCs increased the production of TNF-α, IL-18, IL-6, and IL-23. However, co-treatment with VGX1027 dramatically repressed the production of these cytokines (Figure 6), indicating that VGX-1027 acts on not only macrophages, but also DCs, whose presentation of self-antigens are sufficient to induce autoimmunity in several mouse models (Miller et al., Ann. N.Y. Acad. Sci. (1103) 179-191 (2007); Van Beelen et al., Immunity, (27) 660-669 (2007)).

To determine if this effect could be translated in vivo, mice were injected with LPS and the % of DCs that express CD86, which is a maturation marker, and the MR of CD86 on the positive cells were measured. The activation of DCs via CD86 expression, and not total cytokine levels, was measured in order to ascertain the direct affects of VGX-1027 on DCs. *In vivo* cytokine measurement could be confounded by other cytokine producing cells, such as macrophages. The mice were injected with LPS or LPS+VGX-1027 (VGX-1027 was given 1 hour prior to LPS administration) 3 hours post injection. The spleen from these mice were extracted and analyzed for DCs with CD86 expression. As shown in Figure 7a, LPS injection increased the % of DCs that expressed CD86; and interestingly, VGX-1027 reduced this percentage without affecting the overall percentage of DCs in the spleen (Figure 7a). Moreover, the VGX1027-induced decrease in the percentage of cells that expressed CD86 and was associated with a decrease in the expression of CD86 per cell (Figure 7b). Therefore, VGX-1027 inhibits LPS-induced production of Th17-promoting inflammatory cytokines by DCs *in vitro* and reduces LPS-induced maturation of DCs *in vivo.*

Although inhibition of Th17-promoting cytokine production from DCs is likely to contribute to the decrease of Th17 T cell development, the potential limitation with this mechanism is that other cells may be compensating for the production of Th17- promoting cytokines. Therefore, the direct effect of VGX-1027 was tested on the differentiation of naive CD4 T cells into Th17 T cells. *In vitro* generation of Th17 T cells requires TGF-β, IL-1β, IL-6, and either IL-21 or IL-23. Th17 T cells was assessed with purified CD4 T cells (CD3+ CD4+ CD45RA+ CD25- HLA-DR-) with or without VGX-1027. As shown in Figure 8, an increase in the percentage of T cells that express IL-17, or Th17 T cells, was observed after stimulation with the Th17-promoting cytokine cocktail. A significant increase was observed in the percentage of Th17 T cells with VGX-1027 inclusion during stimulation (Figure 8a).

The decrease in the percentage of Th17 T cells was also associated with a decrease in the amount of IL-17 expressed in a Th17 T cell as measured by mean fluorescent intensity (MFI) (Figure 8b). The decrease in IL-17 was also observed in the culture media (Figure 8c), suggesting that VGX-1027-induced decrease in Th17 T cells leads to a reduction in the secreted form of IL-17.

Considering the experiments in Figure 8 were conducted with purified CD4 T cells, VGX-1027 can exert a negative effect on Th17 T cell development through a direct cell-autonomous effect. This data, coupled with the results from Figure 7, which showed that VGX-1027 treatment inhibits Th17 cytokine production from DCs, provides evidence that VGX-1027 treatment can be sufficient to inhibit the development of Th17 T cells through both cell autonomous (Direct effect on T cells) and non-cell-autonomous mechanisms (Indirect via inhibiting DCs from producing Th17-promoting cytokines).

Based on the above-described in vitro effects of VGX-1027, it is clear that VGX-1027 inhibits TNF-α secretion induced by the activation of most TLRs and IL-1R, and this effect correlates with MyD88-TRAF6-IRAK dependence and inhibition of NF-κβ and p38 pathways. Further, VGX-1027 is not toxic at high concentrations (100mM) in a variety of cell types including HeLa, Jurkat, RD, PBMCs, and U937, and does not inhibit ConA-induced proliferation or INF-γ production of undifferentiated T Cells. Rather, VGX-1027 exhibits specificity toward macrophages and dendritic cells. Lastly, VGX-1027 inhibits the development of Th17 T cells through both cell-autonomous and non-cell-autonomous mechanisms.

### k. VGX-1027 is an Orally-Administered Drug that Effectively Treats a Number of Autoimmune Diseases

Based on the anti-inflammatory data that are presented above, and the promising PK and toxicity data in mice and humans, the efficacy of VGX-1027 was tested as an anti-inflammatory drug in a number of experimental models in mice. The following mouse models of various autoimmune diseases were investigated:
LPS-induced lethality (Endotoxin-induced disease);
pleurisy (Lung inflammation);
chemically induced immunoinflammatory colitis (DNBS model - model of inflammatory bowel disease);
spontaneous and STZ-induced diabetes in NOD mice (Type I diabetes);
type II collagen-induced arthritis (Rheumatoid Arthritis); and
endotoxin-induced uveitis (EIU) in male Lewis rats (model of inflammatory ocular diseases).

Below are the results of the effect of VGX-1027 administration in these mouse models.

### (1) LPS-Induced Lethality (Endotoxin-Induced Shock)

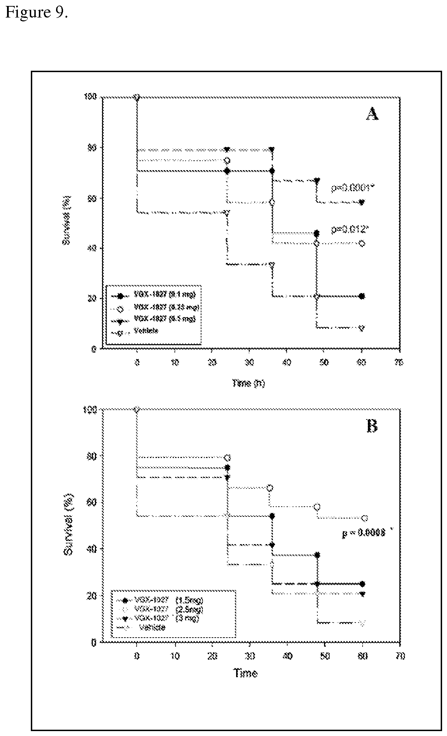

Lipopolysaccharide (LPS) is the most studied of all TLR agonists because it induces both local and systemic inflammation, and responses to LPS mimic many of the features of tissue injury observed during infections with Gram-negative bacteria. Injection of LPS into mice triggers production of inflammatory cytokines and symptoms that are indistinguishable from those of septic shock. As shown in figure 9, VGX-1027 was administered intraperitoneally or orally, and survival was determined after receiving a lethal dose of LPS (1mg/mice) (Figure 9a and b). VGX-1027 treatment increased the survival of mice following LPS injection; this result is consistent with VGX-1027 inhibiting LPS-induced effects on macrophages and DCs. Both intraperitoneal and oral administration of VGX-1027 conferred survival, indicating that oral administration of VGX-1027 is biologically active in repressing cytokine production from LPS.

### (2) Carrageenan (CAR)-induced pleurisy (Lung Inflammation)

Next, carrageenan (CAR)-induced pleurisy, a well-known model of acute inflammation mediated by leukocytes where type-1 proinflammatory cytokines such as IL-1 and TNF-α play a major pathogenic role, was evaluated. Mice that are challenged with CAR develop acute pleurisy with turbid exudates. The amount of inflammation was measured by the volume of exudates, the number of granulocytes (PCN), and the amount of Myeloperoxidase (MPO), activity in the lungs. Anti-TNF antibodies were used as a control to compare the effects of VGX-1027 with those of TNF-α blockade, which is commonly used to treat inflammatory disorders in humans.

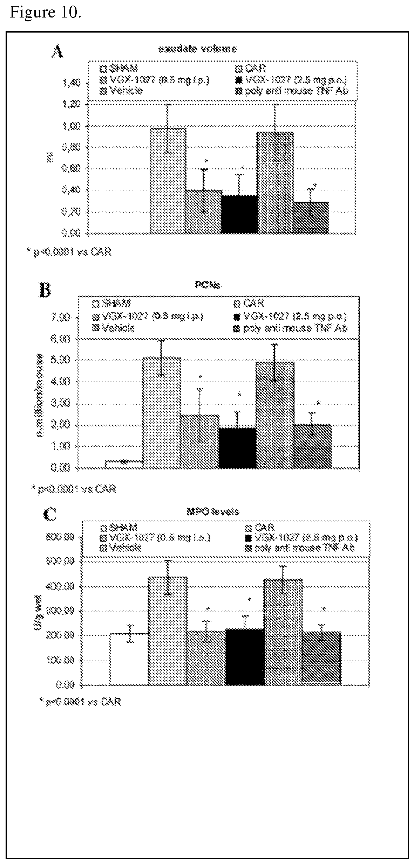

As shown in Figure 10, VGX-1027 treatment effectively decreased all three of the inflammatory measurements induced by CAR. VGX-1027, given either intraperitoneally or orally, was as effective as anti-TNF treatment was in preventing CAR-induced pleurisy.

These laboratory signs of CAR-induced pleurisy were not observed in the sham-treated mice group. Therefore, VGX-1027, in oral form, can be used to treat inflammation of the lung.

### (3) DNBS-Induced Acute Inflammatory Colitis as a Model for Inflammatory Bowel Diseases such as Crohn's Disease and Ulcerative Colitis

Inflammatory bowel disease (IBD) affects approximately 1.8 million people in 7 major markets, with the incidence of Crohn's disease escalating. Ulcerative colitis and Crohn's disease are the 2 primary constituents of IBD and are caused by a failure of the muscosal immune system to attenuate immune responses to self or non-self antigens (Strober et al., J., Clin. Invest., (117) 514-521 (2007)). Therefore, the effect of VGX-1027 on dinitrogenzene sulfonic acid (DNBS)-induced inflammatory colitis was investigated.

DNBS challenge of mice induced clinical features of colitis including weight loss, diarrhea, intestinal damage, and necrotic regions (Figure 11a-d). VGX-1027 treatment, which started 1 hour after DNBS administration and continued until 4 days after DNBS administration, markedly improved a number of features of colitis. This effect was associated with decreased colon weight, an indicator of colon inflammation, and decreased amounts of TNF-α and IL-18 in the colon homogenates (Figure 11e-f). An increase in the weight of the colon is an indirect measure of increased thickness of the walls due to inflammation.

The degree of protection from these clinical and histological signs of colitis afforded by VGX-1027 was comparable to that afforded by 1 mg/kg of dexamethasone, which was used as positive control drug (Figure 11). Therefore, these data indicate that VGX-1027 possesses an immunopharmacological profile that supports its use in the treatment of human inflammatory bowel diseases.

### (4) Spontaneous and STZ-Induced Diabetes in NOD2 Mice as a Model for Type I Diabetes

Type I diabetes mellitus (TID) is a T cell-mediated autoimmune disease that results from selective destruction of the insulin-producing β cells in the pancreatic islets of Langerhans. Both in human TID and in rodent models of the disease, such as MLD-STZ-induced diabetes, the NOD mouse, and the diabetes-prone BB rat, the pancreatic β cells are selectively destroyed by infiltrating mononuclear cells. In addition, Th17 T cells may be involved in the destruction of β cells, and adoptive transfer of Th17 polarized T cells into NOD scid mice is sufficient to transfer TID (Jain et al., J. Exp. Med., (205) 207-218 (2008)). Therefore, the efficacy of VGX-1027 in mitigating the effects of TID in NOD mice and in Streptozotocin (STZ)-induced TID was evaluated.

As shown in Figure 12a, most of the female NOD mice that were not given VGX-1027 developed TID by 25 weeks (13/16 or 81.3% for vehicle; 12/16 or 75% for untreated control). Conversely, prolonged treatment with VGX-1027 reduced the incidence of disease with only 5/16 or 31.3% of mice developing TID. The prolonged treatment - intraperitoneal administration with 20 mg/kg body weight 6 times a week from the 12th week until the 25th week - was well tolerated with no differences in behavior, general appearance, and food consumption.

The effect of VGX-1027 with STZ-induced diabetes was also evaluated. STZ is a DNA alkylating agent with particular toxicity to pancreatic β cells. VGX-1027 was administered orally for 12 days, and STZ was administered into mice for 5 consecutive days starting a day after VGX-1027 treatment. As shown in Figure 12b, STZ alone mice developed hyperglycemia at about ∼2 weeks post STZ injection; conversely, STZ + VGX-1027 mice did not develop hyperglycemia despite only 12 days of VGX-1027 treatment. Moreover, VGX-1027 treatment prevented the infiltration of immune cells and atrophy and loss of islet margins that were observed in the pancreas of STZ alone mice. As these results suggest, VGX-1027 can be used in the prevention and treatment of human TID.

### (5) Endotoxin-induced uveitis (EIU) in rats (Model for ocular inflammatory)

Chronic intraocular inflammation denotes a heterogeneous group of diseases that is a leading cause of acquired blindness in adults. Uveitis refers to inflammation of the uvea, which is the vascular tunic or middle coat of the eye, and is responsible for approximately 3% of blindness in the United States. Unlike other autoimmune conditions with known etiologies, uveitis manifests in a number of inflammatory syndromes including Crohn's disease, Behcet's disease, sarcoidosis, and others (Yeh et al., Semin. Immunopathol., (30) 145-164 (2008)). To study the possible utility of VGX-1027 in treating ocular inflammatory conditions, we injected LPS into footpads of Lewis Rats to induce uveitis. After 16 hours post LPS injection, the rats were sacrificed, and their eyes and aqueous humor were collected to study serological, immunological, and histological signs of EIU. The development of EIU was graded on the following scale: (0) no inflammatory reaction; (1) discrete dilatation of the iris and conjunctival vessels; (2) moderate dilatation of the iris and conjunctival vessels; (3) intense iridal hyperaemia with flare in anterior chamber; and (4) same clinical signs as grade 3 plus the presence of fibrinous exudate in the pupillary area with intense flare in the anterior chamber.

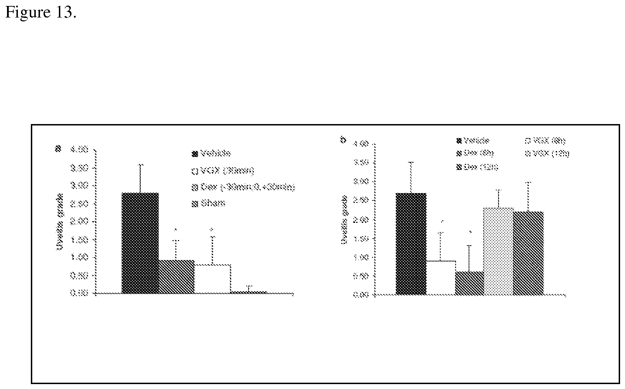

As shown in Figure 13a, the clinical inflammation score with VGX-1027, administered 30 minutes after LPS infection, was significantly lower than that with vehicle alone. A similar decrease in the clinical inflammation grade was observed when VGX-1027 was administered 6 hours post LPS injection; however, the decrease was not observed when VGX-1027 was administered 12 hours post LPS injection. The decrease in the inflammation grade with VGX-1027 treatment was similar to that with dexamethasone treatment. In addition, VGX-1027 also decreased LPS-induced increases in the amount of protein and inflammatory cytokines in the aqueous humor and the infiltration of cells into the iris ciliary body. Therefore, this study demonstrates that VGX-1027 attenuates uveitis induced by LPS in rats, and the effect correlated with a decrease in overall inflammation. These data also suggest that VGX-1027 can be used, perhaps as a topical formulation, to treat or ameliorate human uveitis.

### (6) Type 11 collagen-induced arthritis (Model for Rheumatoid Arthritis)

Rheumatoid arthritis (RA) is a chronic autoimmune disorder that affects a number of tissues and organs, but primarily induces inflammation of the synovial membrane leading to destruction of the articular cartilage and stiffness of the joints. Although the exact mechanism of RA is not completely known, TNF- and IL-1-based biologics appear to attenuate the severity of disease; and like the development of many autoimmune conditions, the development of RA likely requires a host of factors for the appearance of disease (Hirota et al., J. Exp. Med., (204) 41-47 (2007)). One of these factors appears to be Th17 T cells, which VGX-1027 inhibits (Figure 8). IL-17A is found in the synovial fluids of some RA patients and can be detected in T cell-rich areas of RA synovial tissue (Ziolkowsaka et al., J. Immunol., (164) 2823-2838 (2000)). Blockade of IL-17 with anti-IL-17 antibodies reduces joint inflammation, cartilage destruction, and bone erosion in collagen-induced arthritis (CIA), which is the murine model of RA (Lubberts et al., Arthritis Rheum., (50) 650-659 (2004)). Due to the involvement of Th17 T cells in RA, and the effectiveness of anti-IL-1β and TNF-α therapy in ameliorating RA symptoms in humans, the prospects of VGX-1027-based therapy was investigated for treating RA with the aforementioned CIA mouse model.

To induce CIA, the mice were injected intradermally at the base of the tail with 100 pl of an emulsion containing 100 (µl of CII, and were boosted on day 21. In terms of treatment, VGX-1027 was used only as a "therapeutic" regime by administering the drug via intraperitoneal administration to mice with a clinical score equal or higher than 1. The mice were treated with vehicle, dexamethasone (0.3mg/kg), or VGX-1027 (0.5 mg/mouse) for 10 consecutive days. The mice were assessed with the following scoring system: 0=no signs of arthritis; 1=swelling and/or redness of the paw or 1 digit; 2=involvement of 2 joints; 3=involvement of N2 joints, and 4=severe arthritis of the entire paw and digits. An arthritis index was calculated for each mouse by summing the scores for the individual paws. Other clinical severity measures were hind paw volume and histological assessment of damage; the latter was measured with the following criteria: 0=no damage; 1=edema; 2=presence of inflammatory cells; and 3-bone resorption.

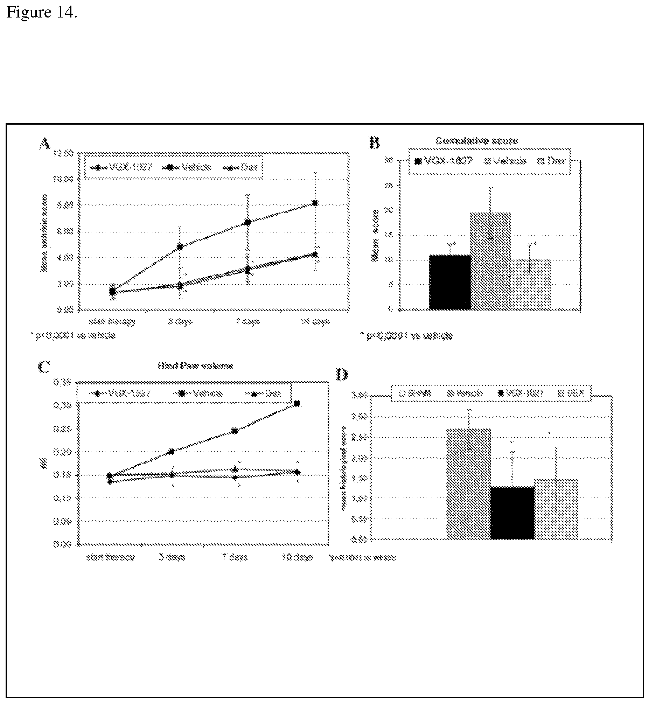

As shown in Figure 14, all of the clinical features of CIA were attenuated with VGX-1027 treatment. Immediate benefits were observed as significant decreases in mean arthritic score and hind paw volume were noted even after just 3 days of VGX-1027 therapy (Figure 14a-c). These effects were also associated with improvements in inflammation via histological measurement (Figure 14d). The amount of improvement in the clinical signs of CIA with VGX-1027 was comparable to the amount observed with dexamethasone (Figure 14). Lastly, these benefits with VGX-1027 were observed after the onset of disease, suggesting that VGX-1027 can be used to ameliorate or assuage RA symptoms in patients.

### l. Extended VGX-1027 Therapy

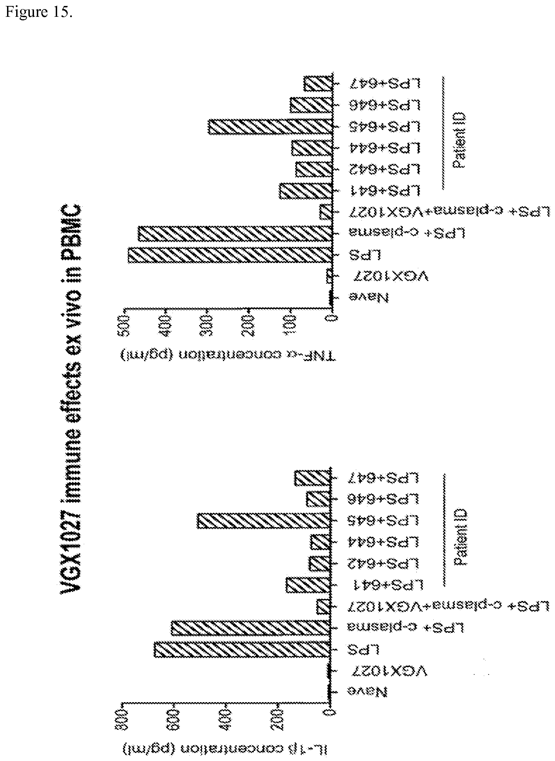

VGX-1027 immune effects *ex vivo* in peripheral blood mononuclear cells (PBMCs) were analyzed to determine whether, and for how long, VGX-1027 could repress or inhibit IL-1β and TNF-α after stimulating the plasma PBMCs with LPS. 5 days after administration of VGX-1027, TNF-α and IL-1β levels were repressed to levels comparable to the negative controls. Compare "LPS + C-plasma + VGX-1027" to "Nave" and "VGX-1027" in Figure 15. This data shows that VGX-1027 therapy may be provided in varying dosing regimens, including regimens that impart extended therapy.

Normal human PBMCs were isolated and cultured in the presence of LPS (5µg/ml) with or without VGX-1027 treated plasma. Plasma samples were obtained by blood collection before and on the 5^{th} day after VGX-1027 dosing in steady state. Cell free supernateants were analyzed for production of TNF-α and IL-1β 24 hours post treatment. Cytokine levels (pg/ml) were assayed by ELISA. Representative results were correlated with pro-inflammatory cytokine inhibition. See Figure 15.

### m. Immune effects of VGX-1027 in two disease models in mice

### (1) The immunomodulatory effects of VGX-1027 in vitro was studied using the genome-wide oligonucleotide microarray approach.

Transcriptional profile analysis: PBMCs from three individual healthy donors, obtained from the University of Pennsylvania School of Medicine, Immunology Clinical Core. Cells (5x10^6) were treated with either LPS (5 µg/ml), LPS (5 µg/ml) + VGX-1027 (10 uM) or PBS for 48 hours and subsequently, RNA was extracted using the RNeasy kit (Qiagen) following manufacturer's instructions.

RNA was hybridized to the Human Gene 1.0 ST array (Affymetrix). Microarray data were analyzed using the web-based utility Expression Profiler (http://www.ebi.ac.uk/expressionprofiler/). Data normalization was achieved by performing logarithmic transformation of all intensities followed by gene mean-centering. Multiple array probes were collapsed by averaging the intensities. Principal component analysis (PCA) was conducted on all genes to assign the general variability in the data to a reduced set of variables. Gene expression differences were assessed by means of Student's t test, and genes with a p<0.01 were considered differentially expressed. Selected genes were subjected to K-means clustering, using Euclidean distance as correlation measurement, and gene ontology (GO) analysis was performed using the Database for Annotation, Visualization and Integrated Discovery (DAVID) tool. Hierarchical clustering of selected genes was performed using Euclidean distance measurements as similarity comparison.

Global transcriptional analysis: The transcriptional changes associated to VGX-1027 treatment upon LPS stimulation were evaluated using oligonucleotide microarrays. Data indicate that VGX-1027 is able to significantly modify the pro-inflammatory events associated to TLR-4 activation. Eigendecomposition yielded eight principal components with four of them associated with most of the co-variability among the data. PCA analysis was performed on the entire dataset and demonstrates that LPS stimulation of PBMCs is associated with a significant modification of the global transcriptome (Figure 16A). VGX-1027 treatment is also associated with dramatic changes at the transcriptional level, and reveals a complex pattern of gene expression. LPS treatment was associated with the modulation of 325 transcripts, while VGX-1027 induced the modulation of 895 genes relative to LPS (Table 3 and Table 4 indicate the top 50 down-regulated and up-regulated genes).

**Table 3. Fifty most down-regulated genes in the VGX-treated group vs. LPS stimulated group**

| **Gene** | **p-value** | **mean/difference of means** | **confidence interval** |
|---|---|---|---|
| 8135943 | 0.0000312 | 0.406 | (0.353, 0.458) |
| CABYR | 0.000213 | 0.135 | (0.109, 0.161) |
| GGTLC2 | 0.000255 | 0.179 | (0.145, 0.213) |
| 8153835 | 0.000303 | 0.0829 | (0.0656, 0.1) |
| LRRC8D | 0.000326 | 0.139 | (0.111, 0.167) |
| EDN2 | 0.00036 | 0.0391 | (0.0295, 0.0487) |
| C11orf53 | 0.000423 | 0.184 | (0.138, 0.229) |
| HAGH | 0.000424 | 0.0712 | (0.0538, 0.0886) |
| TUSC3 | 0.000539 | 0.0955 | (0.0693, 0.122) |
| 8140035 | 0.000735 | 0.388 | (0.273, 0.503) |
| MOV10L1 | 0.000961 | 0.0753 | (0.0519, 0.0988) |
| PAH | 0.00112 | 0.0906 | (0.061, 0.12) |
| EGR4 | 0.00123 | 0.129 | (0.103, 0.155) |
| 8028281 | 0.00134 | 0.0791 | (0.0517, 0.107) |
| SNRNP70 | 0.00137 | 0.0617 | (0.0401, 0.0833) |
| OFCC1 | 0.00139 | 0.0379 | (0.0274, 0.0483) |
| FUK | 0.00141 | 0.0866 | (0.057, 0.116) |
| VIPR1 | 0.00141 | 0.123 | (0.0797, 0.167) |
| YPEL2 | 0.00146 | 0.183 | (0.118, 0.249) |
| TXNRD1 | 0.00152 | 0.117 | (0.0771, 0.157) |
| 8007069 | 0.00153 | 0.074 | (0.0478, 0.1) |
| TRABD | 0.00157 | 0.0439 | (0.0313, 0.0566) |
| UAP1L1 | 0.00174 | 0.0398 | (0.0253, 0.0544) |
| GATAD1 | 0.00179 | 0.0258 | (0.0168, 0.0348) |
| FAM108C1 | 0.00179 | 0.0544 | (0.0369, 0.0718) |
| 8102775 | 0.00186 | 0.101 | (0.068, 0.134) |
| SH3PXD2A | 0.00191 | 0.0515 | (0.0319, 0.071) |
| KLF3 | 0.00203 | 0.115 | (0.0731, 0.157) |
| CCKBR | 0.00227 | 0.0588 | (0.0354, 0.0822) |
| FNBP1L | 0.00231 | 0.203 | (0.125, 0.282) |
| 8094946 | 0.00232 | 0.07 | (0.0424, 0.0977) |
| SLC38A2 | 0.00234 | 0.0979 | (0.0705, 0.125) |
| 7923965 | 0.00241 | 0.439 | (0.262, 0.616) |
| Clorf2 | 0.00279 | 0.106 | (0.0616, 0.151) |
| 8160555 | 0.00299 | 0.171 | (0.101, 0.242) |
| SERPINI1 | 0.00311 | 0.187 | (0.106, 0.267) |
| HSPA12B | 0.0032 | 0.0281 | (0.0174, 0.0388) |
| 7945084 | 0.00322 | 0.165 | (0.102, 0.229) |
| HISPPD2A | 0.00328 | 0.0217 | (0.0123, 0.031) |
| 8134024 | 0.00331 | 0.0796 | (0.0444, 0.115) |
| 8017100 | 0.00338 | 0.108 | (0.0787, 0.138) |
| CYTH1 | 0.0035 | 0.0452 | (0.0272, 0.0632) |
| 8077169 | 0.00362 | 0.153 | (0.0997, 0.207) |
| CIZ1 | 0.00363 | 0.0416 | (0.0232, 0.0601) |
| 7912155 | 0.00372 | 0.111 | (0.0674, 0.154) |
| ALKBH5 | 0.00373 | 0.0698 | (0.0447, 0.0949) |
| ZNF746 | 0.00375 | 0.0386 | (0.0228, 0.0545) |
| ETNK2 | 0.00384 | 0.278 | (0.171, 0.384) |
| 8104068 | 0.00411 | 0.118 | (0.0643, 0.173) |
| C20orf173 | 0.00422 | 0.14 | (0.0766, 0.203) |

**Table 4. Fifty most up-regulated genes in the VGX-treated group vs. LPS stimulated group**

| **Gene** | **p-value** | **mean/difference of means** | **confidence interval** |
|---|---|---|---|
| MAGOH | 9.51E-06 | -0.1 | (-0.109, -0.0911) |
| CD38 | 0.000112 | -0.468 | (-0.519, -0.416) |
| SNORA24 | 0.000134 | -0.212 | (-0.252, -0.172) |
| FCRL1 | 0.000178 | -0.301 | (-0.35, -0.252) |
| GEN1 | 0.000192 | -0.368 | (-0.446, -0.291) |
| DNAH1 | 0.00021 | -0.134 | (-0.16, -0.107) |
| CLIC5 | 0.000255 | -0.175 | (-0.214, -0.136) |
| CPA3 | 4.00E-04 | -0.669 | (-0.831, -0.508) |
| KARS | 0.000424 | -0.126 | (-0.156, -0.0957) |
| PAK1IP1 | 0.000444 | -0.271 | (-0.332, -0.209) |
| CD22 | 0.000527 | -0.453 | (-0.561, -0.344) |
| NLN | 0.000585 | -0.217 | (-0.278, -0.157) |
| SPAG7 | 0.000644 | -0.149 | (-0.188, -0.109) |
| LYRM4 | 0.000663 | -0.294 | (-0.379, -0.21) |
| MRPL15 | 0.000663 | -0.147 | (-0.188, -0.106) |
| CHML | 0.000703 | -0.24 | (-0.31, -0.17) |
| CCL8 | 0.000744 | -1.08 | (-1.3, -0.851) |
| ADAM19 | 0.000748 | -0.176 | (-0.228, -0.123) |
| APOBEC3G | 0.000768 | -0.239 | (-0.307, -0.171) |
| hCG_2024410 | 0.000792 | -0.102 | (-0.133, -0.0714) |
| NDUFA4 | 0.000795 | -0.0638 | (-0.0823, -0.0452) |
| BLNK | 0.000809 | -0.398 | (-0.518, -0.277) |
| 8043441 | 0.000815 | -0.406 | (-0.527, -0.285) |
| EOMES | 0.000821 | -0.0887 | (-0.116, -0.0616) |
| BOLA3 | 0.000826 | -0.118 | (-0.149, -0.0869) |
| SRFBP1 | 0.00104 | -0.124 | (-0.163, -0.0841) |
| WARS2 | 0.00108 | -0.246 | (-0.325, -0.168) |
| ATXN10 | 0.0011 | -0.131 | (-0.17, -0.0921) |
| OAS3 | 0.00111 | -0.134 | (-0.178, -0.0896) |
| WDR70 | 0.00111 | -0.0908 | (-0.114, -0.0673) |
| LCP2 | 0.00114 | -0.0536 | (-0.0711, -0.0361) |
| 8136446 | 0.00116 | -0.146 | (-0.194, -0.0983) |
| AIM2 | 0.00117 | -0.221 | (-0.285, -0.156) |
| RGS1 | 0.00118 | -0.294 | (-0.388, -0.2) |
| 7997245 | 0.00129 | -0.0599 | (-0.0799, -0.04) |
| CLU | 0.00131 | -0.218 | (-0.283, -0.153) |
| LARS2 | 0.00138 | -0.176 | (-0.237, -0.114) |
| MRPS35 | 0.00142 | -0.164 | (-0.222, -0.106) |
| KLHDC4 | 0.00147 | -0.0727 | (-0.0967, -0.0487) |
| TCL1A | 0.00152 | -0.442 | (-0.534, -0.351) |
| 8150101 | 0.00152 | -0.237 | (-0.322, -0.152) |
| FAS | 0.00153 | -0.12 | (-0.154, -0.086) |
| PGDS | 0.00155 | -0.708 | (-0.895, -0.521) |
| KCTD10 | 0.00159 | -0.0613 | (-0.081, -0.0416) |
| KLF10 | 0.00161 | -0.11 | (-0.141, -0.0795) |
| SNX6 | 0.00162 | -0.0607 | (-0.0823, -0.039) |
| ZNF596 | 0.00164 | -0.432 | (-0.591, -0.274) |
| TSN | 0.00168 | -0.105 | (-0.142, -0.069) |
| RPE | 0.0017 | -0.116 | (-0.159, -0.0731) |
| TNFRSF17 | 0.00174 | -0.295 | (-0.404, -0.186) |

GO analysis was performed using the Kyoto Encyclopedia of Genes and Genomes (KEGG) Database (Kanehisa et al., 2012). An overrepresentation of molecules associated to "Proteasome", "Antigen Processing and presentation", "Spliceosome" and "Citrate Cycle" were found (Table 5).

**Table 5. GO analysis of differentially expressed genes**

| **Term** | **Count** | **P value** | **Fold Enrichment** | **Benjamini** | **FDR** |
|---|---|---|---|---|---|
| hsa03050:Proteasome | 13 | 1.22E-06 | 5.78802 | 1.71E-04 | 0.00144 |
| hsa04612:Antigen processing and presentation | 13 | 4.89E-04 | 3.27755 | 0.03388 | 0.57584 |
| hsa03040:Spliceosome | 16 | 8.47E-04 | 2.65726 | 0.03904 | 0.99599 |
| hsa00020:Citrate cycle (TCA cycle) | 7 | 0.00295 | 4.72521 | 0.09875 | 3.42467 |
| hsa00240:Pyrimidine metabolism | 12 | 0.00509 | 2.64327 | 0.13413 | 5.855 |
| hsa04110:Cell cycle | 14 | 0.0061 | 2.3437 | 0.13402 | 6.97828 |
| hsa00230:Purine metabolism | 15 | 0.01354 | 2.05156 | 0.24006 | 14.8713 |
| hsa03022:Basal transcription factors | 6 | 0.02363 | 3.5873 | 0.34393 | 24.6119 |
| hsa00970:Aminoacyl-tRNA biosynthesis | 6 | 0.0434 | 3.06233 | 0.50096 | 40.7927 |
| hsa03018:RNA degradation | 7 | 0.05222 | 2.56985 | 0.53053 | 46.9296 |
| hsa05012:Parkinson's disease | 11 | 0.08262 | 1.79832 | 0.66889 | 63.8937 |
| hsa05322:Systemic lupus erythematosus | 9 | 0.09778 | 1.90236 | 0.70153 | 70.3483 |

Differentially expressed genes were subjected to K-means clustering performed for a total of 5 clusters. Euclidean distance was used as similarity measurement and initialized by the most distant genes (Figure 16B). To gain insight into the biological processes involved, GO analysis was performed on each cluster. One cluster included 525 genes, which were strongly downregulated upon treatment with VGX1027 but only partially upregulated by LPS. GO analysis revealed enriched sets of genes belonging to the "Proteasome", the "Spliceosome" and the "Antigen Processing and Presentation" pathways. A third cluster of genes included genes which were downregulated upon LPS stimulation and further downregulated by VGX1027 (CD180, TLR7, MS4A6A, FCGR3A, CX3CR1, ENPP2, NLN, C17orf87). One more cluster of genes, included transcripts strongly upregulated by LPS which returned to basal levels upon administration of VGX1027 (IL12RB2, RGS1, CCL7, RSAD2, IL22, IFNG). Finally, two clusters of 61 and 296 gene were found to be significantly related to the KEGG Categories "Cell Cycle" and "Basal Transcription Factors" (STON1-GTF2A1L, LOC391764, GTF2IRD1), respectively.

### GO analysis of functional categories: "Antigen Processing and Presentation"

The genes associated to the "Antigen Processing and Presentation" pathway belonged to the family of the Heat Shock Proteins (HSP90AB1 and HSPA8), which are closely involved in the folding of peptides to be loaded onto the MHC proteins; the MHC class II molecules HLA-DOA and HLA-DRA; the transmembrane glycoprotein TAPBP, which mediates the interaction between newly assembled MHC class I molecules and TAP, and that is required for the transport of antigenic peptides across the endoplasmic reticulum membrane; the Killer Cell Immunoglobulin-like Receptors KIR3DS1, KIR2DS1, KIR2DS4 and KIR3DL1, which are involved in the transduction of the activation signals in NK cells and T cells; the protein disulfide isomerase PDIA3, that modulates the folding of newly synthesized glycoproteins; and finally the genes that encodes for the three subunits of the 11S regulator of PA28 which is the endogenous regulator of the immunoproteasome, involved in the processing of class I MHC peptides (Figure 16C).

### GO analysis of functional categories: "Cell Cycle"

A second category highlighted by GO analysis was represented by "Cell Cycle". The genes correlated to this category were represented by the two cyclin family members, CCNE2 and CCNB1, which are predominantly expressed in the G1/S and G2/M transition, respectively. Two other genes were CDC6 and ORC6, involved in the initiation of DNA replication and in coordinating chromosome replication and segregation with cytokinesis. Downregulation of the genes BUB1 and BUB1B was also observed in the VGX-1027 treated cells in comparison to the LPS group. These two transcripts are essential for spindle-assembly checkpoint signaling and for correct chromosome alignment. CHK1 and MCM4 are involved in the regulation of cell cycle progression and the initiation of eukaryotic genome replication, respectively (Figure 16D).

In vitro T-cell treatment: Naive, sorted CD4+ T cells were obtained from the University of Pennsylvania School of Medicine, Immunology Clinical Core, cultured in vitro with IL-6 (20 ng/ml) in the presence or absence of VGX1027 (10 uM) for 0,30 min, 1hr, 2hrs and 5 hrs. At the end of the incubation period, cells were lysed for 30 min at 4°C by using the Nonidet P-40 lysis buffer (Invitrogen) supplemented with a phosphatase inhibitor mixture (PhosSTOP) and a protease inhibitor mixture (Complete) as directed by the manufacturer (Roche Applied Science). Lysates were collected and spun at 10,000 rpm for 5 min at 4°C, and then, 50 ug of proteins were mixed with 2X sample buffer (Invitrogen) for subsequent 4-12% SDS-PAGE electrophoresis. Afterward, proteins were transferred to nitrocellulose using the iBlot Dry Blotting System (Invitrogen), immunodetection was performed with the SNAP i.d. Protein Detection System (Millipore) using specific p-STAT3 and STAT3 monoclonal antibodies (Cell Signaling), and the expressed proteins were visualized with HRP-conjugated goat antimouse IgG using an ECL detection system (Amersham Biosciences, Piscataway, NJ).

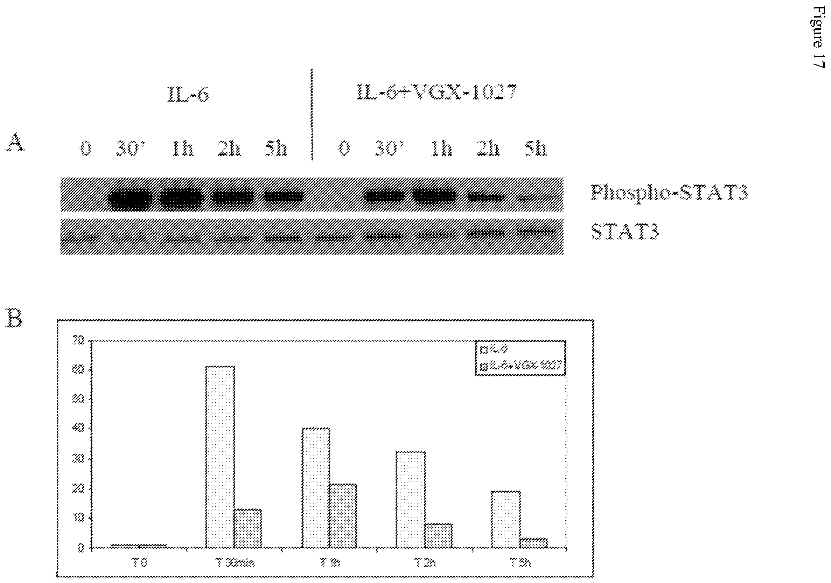

CD4+ T cells were exposed for increasing periods of time to IL-6 and were either treated with VGX-1027 or with vehicle. It is known that IL-6, upon engagement with the gp130 receptors, induces the phosphorilation of STAT3, after the activation of the JAK2 kinase (Murray PJ. 2007. The JAK-STAT signaling pathway: Input and output integration. J Immunol 178:2623-2629.). The effects of VGX-1027 in vitro on the relative levels of p-STAT3 were determined by Western blot after stimulation of naive T helper cells to IL-6. The levels of activated STAT3 reached a peak around 30 min after IL-6 stimulation, then declined afterward in a time-dependent manner (Figure 17). The administration of VGX-1027 was associated to an overall reduction in the magnitude of STAT3 phosphorilation. The peak of activation was delayed, reaching the maximum 1 h after IL-6 administration. The above results show that VGX-1027 is able to significantly affect the JAK/STAT signaling pathway induced by the pro-inflammatory cytokine IL6 (Figure 17).

### (2) The immunomodulatory effects of VGX-1027 in vivo was studied using two different murine models of immunological diseases: the staphilococcus aureus enterotoxin B (SEB)-induced shock and the NZB/NZW F1 model of systemic lupus erythematosus (SLE).

SLE is a complex autoimmune disease with heterogeneous clinical manifestations and course. Current treatment includes the use of anti-inflammatory drugs, antimalarial agents and immunosuppressive drugs, including azathioprine, cyclophosphamide, methotrexate and mycophenolic acid. However, despite the significant improvement in the prognosis of SLE patients, less toxic pharmacological agents are warranted. Moreover, refractory disease represents a huge issue with no new therapy approved for the treatment of SLE in the past fifty years.

### Protection of mice from SEB-induced lethal shock by VGX-1027

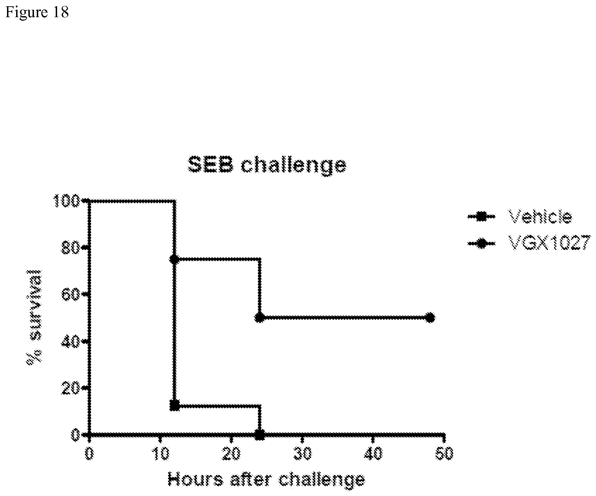

The protective effects of VGX-1027 were investigated in the SEB-induced lethal shock model. Challenged mice started to exhibit disease symptoms (matted fur) within 1 h post-challenge. One hundred percent of vehicle-treated mice exposed to 10 ug dose of SEB died in a period of 24 hrs with 90% of them being dead in the first 12 hrs. The administration of a single dose of VGX-1027, 30 min after SEB injection, was mostly effective at preventing toxin-induced death. Survival was improved in SEB-challenged mice from 0 to 50% in the VGX-1027 treated animals (p=0.007 by Logrank test). Moreover, VGX-1027 treatment attenuated symptomology associated with SEB challenge. However, a minority of animals still died from the SEB challenge, although, these deaths occurred later than 24 hrs after all the vehicle treated animals succumbed (Figure 18).

Effects of VGX-1027 in a murine model of SLE / Effects of VGX-1027 on proteinuria and survival of NZB/NZW F1 mice

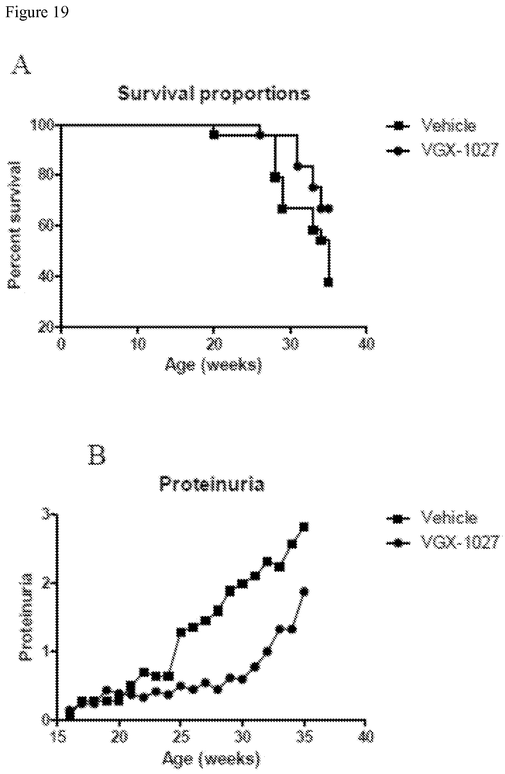

To determine the effects of VGX-1027 on the development and progression of SLE, lupus-prone NZB/NZW F1 were treated daily with 5 mg/mouse VGX-1027 i.p. for 20 weeks, starting at 16 weeks of age. Control mice received an equal volume of vehicle. Control mice started to die at 20 weeks of age. By week 36, 62.5% of them were dead compared to the only 33.3% of treated mice (p=0.0487 by Log-rank test) (Figure 19A). Improved survival was correlated with inhibition of renal disease. Proteinuria was assessed once a week. The administration of VGX-1027 significantly improved renal disease and the levels of proteinuria in VGX-1027 treated mice remained significantly lower throughout the experimental period (p=0.0265 by Student t test). Eventually, all of the mice developed proteinuria but the delay in disease progression and prolonged survival of VGX-1027 treated animals remained evident (Figure 19B).

### VGX-1027 reduced anti-dsDNA autoantibody production and nephritis

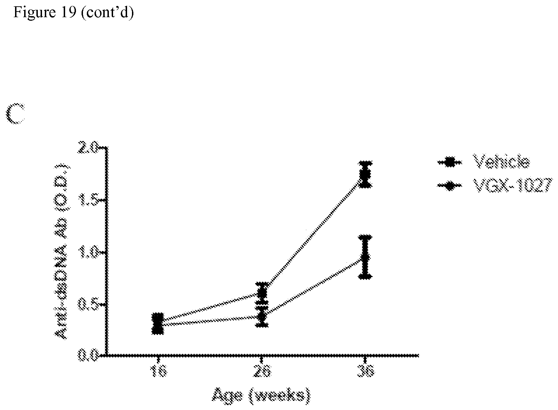

The presence of anti-dsDNA Abs is commonly used as a biomarker associated with poor prognosis of SLE and is strongly associated with developing lupus nephritis. The effect of VGX-1027 on anti-dsDNA autoantibody production was evaluated after 10 weeks of treatment and at the end of the experimental period. Only slightly increased levels of anti-dsDNA antibodies were found at 26 weeks of age in both vehicle and VGX-1027 treated animals. In contrast, at the termination of the study, when mice were 36-weeks old, VGX-1027 treated mice showed significant lower levels of autoantibodies in the serum (p<0.0001 by ANOVA) (Figure 19C).

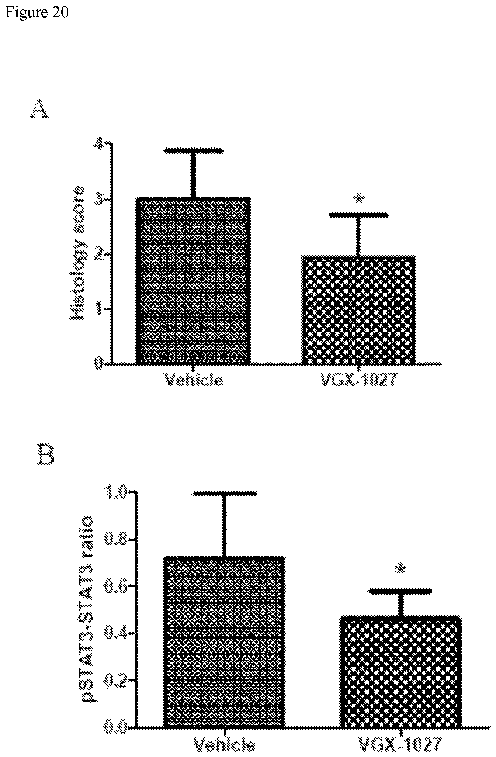

End-stage SLE nephritis was evaluated by histopathology and scored by an indipendent pathologist for the assessment of total renal damage. Decrease in cellular infiltration/glomerulonephritis were observed in the H&E-stained renal sections from the VGX-1027 treated group compared with the vehicle treatment group. Mean scores were 1.9±0.8 and 3.1±0.9 for the VGX-1027 and the vehicle control group, respectively (p=0.01 by Mann Whitney U test) (Figure 20A).

Significant reductions in renal p-STAT3 was assessed by ELISA in order to evaluate the effects of VGX-1027 on the IL-6/JAK/STAT signaling pathway. VGX-1027 treatment was associated with significant reduction (p=0.0234 by Student t test) of the relative levels of p-STAT3. Thus, treatment of NZB/NZW F1 mice with VGX-1027 is able to delay the development of SLE nephritis, partially blocking the IL-6 induced proinflammatory pathway (Figure 20B).

Studies both in vitro and on these murine models with the VGX-1027 compound showed the compound can reduce the secretion of both the pro-inflammatory cytokines IL-1β, and TNF-α, and the anti-inflammatory cytokine IL-10 from murine macrophages stimulated in vitro with LPS. It also showed the ability to down-regulate the activation of the NF-κB and p38 MAP kinase pathways along with an up-regulation of the ERK pathway. Further, the genome-wide microarray approach was used to investigate the overall effect of VGX-1027 on a mixed population of immune cells upon exposure to the TLR-4 ligand, LPS. Microarray data revealed that the administration of VGX-1027 significantly affected the immune response to exogenous antigens, by strongly modulating the expression of genes which are primarily involved in the antigen processing and presentation as well as genes which regulate cell cycle progression and proliferation. The data obtained are consistent with additional data from experiments herein, which revealed that VGX-1027 inhibits both proliferation of enterobacterial antigen-reactive CD4+CD25- T cells in vitro and the development of clinical and histological signs of colitis in vivo. The beneficial effect in the DNBS-induced acute inflammatory colitis model was found to be associated with reduced colonic production of proinflammatory cytokines such as interleukin (IL)-1β, TNF-α, IL-12p70 and interferon (IFN)-γ and lower content of nuclear factor (NF)-κB (p65) (Mangano K, Sardesai N, D'Alcamo M, Libra M, Malaguarnera L, Donia M, Bendtzen K, Meroni P, Nicoletti F. In vitro inhibition of enterobacteria-reactive CD4+CD25-T cells and suppression of immunoinflammatory colitis in mice by the novel immunomodulatory agent VGX-1027. Eur J Pharmacol. 2008 May 31;586(1-3):313-21).

VGX-1027 was also shown to affect the JAK/STAT pathway induced by IL-6. The compound was shown to be able to strongly reduce the activation of STAT3, a kinase downstream the gp130 receptors. STAT3 signaling appears to regulate the survival and activation of T cells and the differentiation of B-cell into plasma cells; it also plays a critical role for antigen-presenting cell-mediated T-cell activation and tolerance. IL-6 represents one of the major contributors for STAT3 activation, and it is implicated in the etiopathogenesis of various autoimmune diseases given its wide range of target cell in inflammation. In SLE, infiltrating macrophages into the kidney are the main source of IL-6. High concentrations of IL-6 can be found in sera of SLE patients as well as in murine SLE models. Blocking IL-6 with anti-IL-6 antibodies has been found to reduce nephritis in the lupus-prone NZB/NZW F1 mouse. The administration of the VGX-1027 compound significantly increased survival of NZB/NZW F1 mice and reduced kidney pathology, as shown by the proteinuria levels and histopathology. Moreover, reduced levels of phosphorilated STAT3 were found in the kidneys of the surviving animals at the end of the experimental period. Also, a significant lower percentage of mice challenged with SEB died when therapeutically treated with VGX-1027. The data presented herein demonstrate that VGX-1027 is able to orchestrate the immune responses by modulating genes produced by different immune cell types at multiple levels and, at least partially, by affecting the ability of cells to promote antigen presentation and/or cell proliferation.

### n. VGX-1027 - Disease Targets, Comparisons with other Compounds, and Mechanism Advantages

On the basis of the experiments and data provided above, VGX-1027 may be used to repress or inhibit the production of IL-18, IL-6, IL-23, and/or TNF-α. Dendritic cells make these cytokines. Th17 cell production *in vitro* requires the cytokines IL-6, IL-21 or IL-23, and TGF-β and the transcription factor RORyt. Because hyperactive and/or the production of Th17 T cells can induce most autoimmune disorders, VGX-1027 may be used to treat, for example, autoimmune uveitis, lung inflammation, inflammatory bowel diseases, rheumatoid arthritis, and/or type I diabetes. VGX-1027 may be used to treat the correlated disease or disorder as this drug regulates cytokines IL-18, IL-6 and IL-23, which are often used as markers for various diseases and disorders. For example, increased levels of IL-18 have been found in subjects having coeliac disease, periodontal disease and Crohn's disease. See, for example, Lettesjo, et al., Clinical and Experimental Immunology, 139:138-143 (2005); Orozco, et al., J. of Dental Research, 86(7): 586-593 (2007); and Pizzaro et al., J. of Immunology, 162:6829-6835 (1999). Increased levels of IL-6 have been associated with Type II diabetes, plasmacytosis, rheumatoid arthritis, systemic-onset juvenile chronic arthritis, osteoporosis, psoriasis, autoimmune disease, such as antigen-induced arthritis, experimental allergic encephalomyelitis, inflammatory bowel disease, Castleman's disease, and, in general, autoimmune disease and chronic inflammatory proliferative diseases. See, for example, Hu et al., Diabetes, 53(3): 693-700 (2004); Nishimoto et al., Blood, 95:56-61 (2000); Ishihara et al., Cytokine Growth Reviews, 13(4-5): 357-368 (2002); Ito, Curr. Drug Targets Inflamm. Allergy, 2(2): 125-130 (2003); and Mudter et al., Inflammatory Bowel Diseases, 13(8): 1016-1023 (2007). Increased levels of IL-23 have been found to be associated with, for example, autoimmune inflammation, such as autoimmune encephalomyelitis. See, for example, Chen et al., J. Clin. Invent., 116(5): 1317-1326 (2006).

Numerous anti-inflammatory drugs, which include p38 and NF-κB inhibitors, have demonstrated efficacy in preclinical models, but hurdles related to toxicity and/or efficacy -have precluded their advancement. Classic examples include Vertex and Boehringer Ingelheim's p38 inhibitors, which have been discontinued in Phase II due to liver toxicity (Reviewed in Dambach, Curr. Top. Med. Chem., (5) 929-939 (2005)). While VGX-1027's clinical potential remains to be determined, VGX-1027 appears to be safe and well-tolerated at even the highest dose tested in humans. In addition, we propose several crucial differences between VGX-1027 and these other drugs.

In contrast with classical p38 inhibitors, VGX-1027 appears to selectively inhibit the activation of antigen presenting cells and the development of TH 17 cells. Treatment of APCs with VGX-1027 inhibits LPS-induced TNF-α/IL-1β production, CD80/86 up-regulation, and APC-mediated T cell activation. Further, treatment of T cells with VGX-1027 prevents the development of Th17 T cells (Figures 4-8). Conversely, VGX-1027 does not appear to affect ConA and CD3/CD28-induced T cell proliferation. Thus, VGX-1027 could contribute ostensibly to minimizing undesirable toxicity (Figure 5a). This is in contrast with p38 inhibitors, which inhibit the proliferation of T cells indiscriminately (Ward et al., Biochem. Soc. Trans., (25) 304S (1997); Crawley et al., J. Biol. Chem., (272) 15023-15027 (1997)). However, this selectivity appears unlikely to limit VGX-1027's anti-inflammatory potential, because in many cases, such as insulin resistance, Duchenne muscular dystrophy, and certain models of chronic inflammation, myeloid-specific inhibition of cytokine-induced kinases is sufficient to reverse the respective condition (Arkan et al., Nat. Med., (11) 191-198 (2005); Acharyya et al., J. Clin. Invest., (117) 889-901 (2007); Eckmann et al., Proc. Natl. Acad. Sci. USA, (105) 15058-15063 (2008)).

Furthermore, the fact that VGX-1027 is not a direct inhibitor of enzymes such as p38 and IKKβ is likely an advantage in terms of minimizing toxicity. Genetic experiments have shown that these enzymes are necessary for basic physiological functions. Loss of NF-KB function leads to embryonic lethality associated with massive liver degeneration, and hepatocyte-specific loss of p38α cooperates with NF-κB inhibition to lead to endotoxin-induced liver damage (Beg et al., Science, (274) 782-784 (1996); Li et al., J. Exp. Med. (189) 1839-1845 (1999); Heinrichsdorff et al., EMBO Rep., (9) 1048-104 (2008)). However, VGX-1027, unlike the other drugs, would only inhibit NF-κB and p38 in inflammatory contexts where the activation is driven by TLRs or IL-1R. Therefore, the key is inhibiting these pathways specifically in cells that are responsible for inflammation without inhibiting these same pathways in bystander cells. This criterion is not achieved with traditional p38 and NF-κB inhibitors, and thus may contribute to undesirable toxicity such as elevated plasma transaminase levels (Dambach et al., Curr. Top. Med. Chem. (5) 929-939 (2005)).

In one aspect, VGX-1027's can target LPS-induced, but not poly(I:C)-induced, macrophage activation and TNF-α production (Figure 5). This suggests that VGX-1027's effect is not only at the level of cell type specificity, but also at pathway specificity, considering LPS requires TLR-4 and poly(I:C) requires TLR-3. VGX1027's effect correlates with MyD88-IRAK dependence, although more investigation is warranted (Figure 2 & 4). Therefore, one would not expect VGX-1027 to inhibit the activities of p38 and NF-κB globally, but rather inhibit the activities in specific cells and/or situations (Stojanovic et al., Clin. Immunol., (123) 311-323 (2007); Mangano et al., Br. J. Pharmacol., (155) 722-730 (2008)). As a result, VGX-1027 is anticipated to exhibit less toxicity than the other aforementioned drugs do, but with pathway-specific anti-inflammatory properties.

The data provided herein suggests that VGX-1027 inhibits inflammation through at least two distinct mechanisms, although both may require the inhibition of the same target protein. First, VGX-1027 inhibits the activation of APCs as measured by decreased cytokine production and maturation. However, this effect was restricted to stimulation with agonists - LPS, IL-1β, ZYM, and MDP - that required the MyD88-IRAK pathway (Figure 2 and 4). Second, VGX-1027 inhibits the development of Th17 T cells through both cell-autonomous and non-cell-autonomous mechanisms (Figure 6 & 8). There are several advantages to this mechanism. A number of conditions require the inhibition of multiple factors for reduction of clinical symptoms. For example, a third of Crohn's disease patients do not respond to anti-TNF therapy. By targeting the production of numerous cytokines, including TNF-α and IL-1β, VGX-1027 treatment would be sufficient to mitigate this limitation. Further, most autoimmune disorders are now believed to be driven by hyperactive and/or production of Th17 T cells. These cells are highly abundant in the tissues of patients suffering from autoimmune disorders and are necessary for a number of autoimmune disorders in mice. Currently, there are no drugs in the market that specifically inhibit Th17 T cells, although anti-p40 biologics (ABT-874 and CNTO-1275), which target the p40 subunit of both IL-12 and IL23, are awaiting approval in the US (Reich et al., Nat. Rev. Drug. Discov., (8) 355-356 (2009); Lima et al., Expert. Opin. Biol. Ther. (9) 1107-1113 (2009)). Conversely, there are no direct small molecule inhibitors of Th17 T cells, and only indirect measures by inhibiting MAPKs and FLT3 of DCs and APCs (Brereton et al., J. Immunol., (183) 1715-1723 (2009); Skarica et al., J. Immunol., (182) 4192-4199 (2009)). The data described herein suggest that VGX-1027 inhibits not only the APC-mediated effect, but also the direct differentiation process of Th17 T cells (Figure 6 & 8). Therefore, VGX-1027 warrants consideration in treating a host of autoimmune disorders because of its novel mechanism of inhibiting APC activation and Th17 T cells development.

### o. VGX-1027 - Dosing studies in humans for safety

A placebo-controlled, ascending single-dose study was performed to evaluate the safety and pharmacokinetics of VGX-1027 in healthy human subjects. Study was performed upon 7 groups of subjects: group 1: 1 mg of VGX-1027 after fasting; group 2: 10 mg of VGX-1027 after fasting; group 3: 100 mg of VGX-1027 after fasting; group 4: 200 mg of VGX-1027 after fasting; group 5: 400 mg of VGX-1027 after fasting; group 6: 800 mg of VGX-1027 after fasting; and group 7: 100 mg of VGX-1027 after fasting, and after fed. The pharmacokinetics is provided in Table 6, below:

**Table_6**

| **Dose^{a} (mg)** | **Tmax^{b} (hr)** | **Cmax^{c} (ug/mL)** | **AUC^{c} (ug*h/mL)** | **Half-life^{c} (hr)** |
|---|---|---|---|---|
| 1 | 1.00 | 0.05 | 0.46 | 7.2 |
| 10 | 0.50 | 1.1 | 4.43 | 4.9 |
| 100 | 1.75 | 9.1 | 74.6 | 8.8 |
| 200 | 1.50 | 15.7 | 111.1 | 7.7 |
| 400 | 1.25 | 23.8 | 155.2 | 6.7 |
| 800 | 2.00 | 43.2 | 329.7 | 7.9 |

| Fasted vs Fed | | | | |
|---|---|---|---|---|
| 100 fasted | 1.25 | 6.5 | 37.8 | 5.9 |
| 100 fed | 3.50 | 4.6 | 55.7 | 6.3 |

| | | | | |
|---|---|---|---|---|
| ^{a.} administered fasted ^{b} median ^{c} Geometric mean Comparison of geometric mean profiles across dose levels can be seen in Figure 21. | | | | |

A placebo-controlled, ascending multiple-dose study was also performed to evaluate the safety and pharmacokinetics of VGX-1027 in healthy human subjects. The primary outcome to be measure was the safety as determined by vital signs, physical examinations, ECGs, clinical laboratory evaluations, and adverse event reporting. The secondary outcome measured was the pharmacokinetics on day 1 and at steady state-on day 5. Study was performed upon 4 groups of subjects: group 1: 40 mg of VGX-1027 QD; group 2: 100 mg of VGX-1027 QD; group 3: 200 mg of VGX-1027 QD; group 4: 200 mg of VGX-1027 BID. The pharmacokinetics is provided in Table 7, below, along with data from the single dose study:

**Table 7**

| **Dose** | **Single Dose (CAT001)** | | | **Multiple Dose (Day 1)** | | | **Multiple Dose (Day 5)** | | |
|---|---|---|---|---|---|---|---|---|---|
| | **Tmax (hr) | Cmax (mg/L) | AUC (0-24) (hr*mg/L) | **Tmax (hr) | Cmax (mg/L) | AUC (0-24) (hr*mg/L) | **Tmax (hr) | Cmax (mg/L) | AUC (0-24) (hr*mg/L) |
| 100mg | | | | | | | | | |
| Geo Mean | 1.8 | 9.07 | 66.83 | 1.0 | 7.57 | 31.34 | 1.5 | 6.76 | 23.23 |
| Geo CV% | 0.5 - 2.0 | 29.03 | 61.75 | 0.5 - 3.0 | 30.71 | 21.25 | 0.5 - 3.0 | 21.62 | 31.15 |

| 200mg | | | | | | | | | |
|---|---|---|---|---|---|---|---|---|---|
| Geo Mean | 1.5 | 15.69 | 98.41 | 1.3 | 15.74 | 78.10 | 2.0 | 14.07 | 65.67 |
| Geo CV% | 1.0 - 4.0 | 37.2 | 32.10 | 1.0 - 3.0 | 38.40 | 65.77 | 0.5 - 3.0 | 29.97 | 47.65 |

| 200 vs first dose BID on Day 1 and Day 5 (AUC to 12 hours) | | | | | | | | | |
|---|---|---|---|---|---|---|---|---|---|
| Geo Mean | 1.5 | 15.69 | 78.54 | 1.0 | 13.10 | 58.22 | 1.5 | 16.80 | 70.39 |
| Geo CV% | 1.0 - 4.0 | 37.2 | 30.65 | 0.5 - 3.0 | 24.60 | 24.46 | 0.5 - 3.0 | 30.61 | 35.88 |

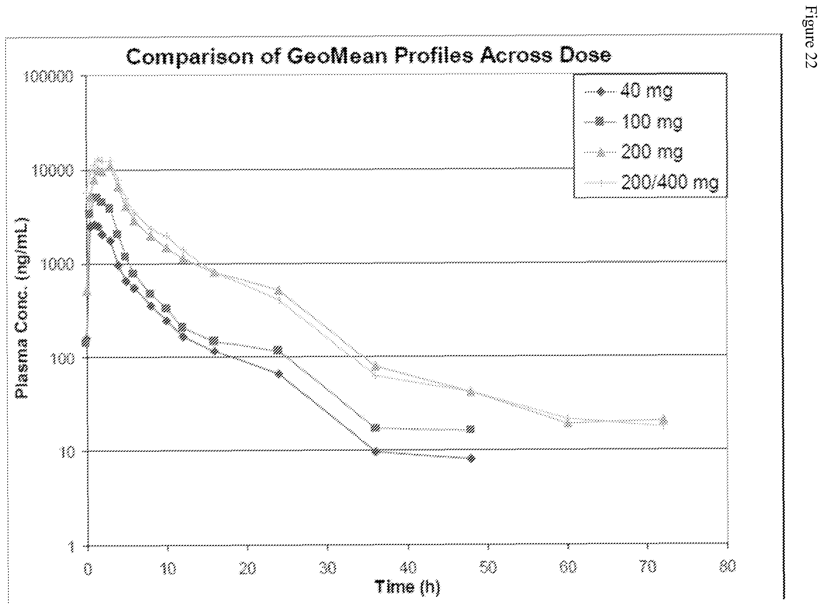

Comparison of geometric mean profiles across dose levels can be seen in Figure 22.

## Claims

1. A compound, wherein the compound is 3-phenyl-4,5-dihydro-5- isoxazoleacetic acid represented by the formula: 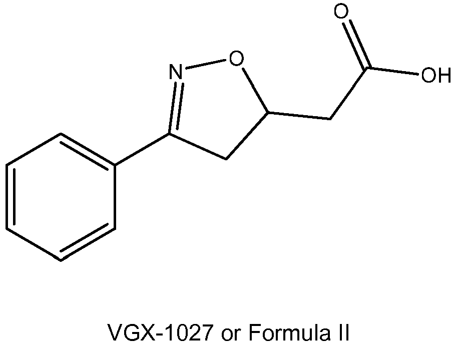 for use in a method of treating autoimmune encephalomyelitis or systemic lupus erythematosus.

2. A pharmaceutical composition comprising the compound of claim 1 for use in a method of treating autoimmune encephalomyelitis or systemic lupus erythematosus.

## Patentansprüche

1. Verbindung, wobei es sich bei der Verbindung um 3-Phenyl-4,5-dihydro-5-isoxazolessigsäure, wiedergegeben durch die Formel: 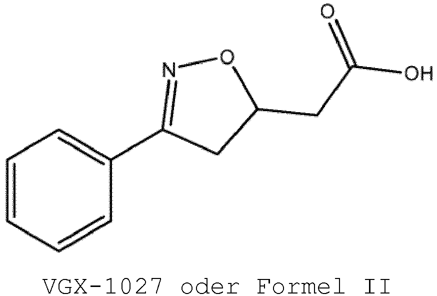 handelt, zur Verwendung in einem Verfahren zur Behandlung von Autoimmunenzephalomyelitis oder systemischem Lupus erythematodes.

2. Pharmazeutische Zusammensetzung, umfassend die Verbindung nach Anspruch 1, zur Verwendung in einem Verfahren zur Behandlung von Autoimmunenzephalomyelitis oder systemischem Lupus erythematodes.

## Revendications

1. Composé, le composé étant l'acide 3-phényl-4,5-dihydro-5-isoxazolacétique représenté par la formule : 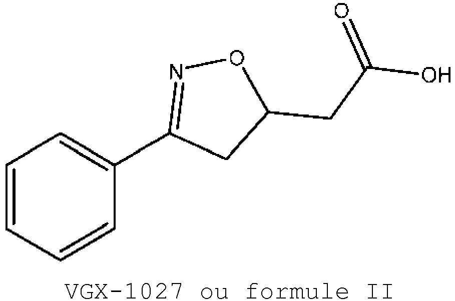 pour utilisation dans un procédé de traitement de l'encéphalomyélite auto-immune ou du lupus érythémateux disséminé.

2. Composition pharmaceutique comprenant le composé selon la revendication 1 pour utilisation dans un procédé de traitement de l'encéphalomyélite auto-immune ou du lupus érythémateux disséminé.
